(19) Europäisches Patentamt
European Patent Office
Office européen des brevets

(11) **EP 4 280 225 A2**

(12) **EUROPEAN PATENT APPLICATION**

(43) Date of publication:
**22.11.2023   Bulletin 2023/47**

(51) International Patent Classification (IPC):
**G16H 50/30** (2018.01)

(21) Application number: **23202274.9**

(22) Date of filing: **25.07.2017**

(52) Cooperative Patent Classification (CPC):
**G16H 20/70; G16H 50/30;** G16H 20/10;
G16H 20/30; G16H 20/60; G16H 50/70

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.07.2016   GB 201612842**

(62) Document number(s) of the earlier application(s) in accordance with Art. 76 EPC:
**17777941.0 / 3 488 369**

(71) Applicant: **Fitnessgenes Limited
Bicester, Oxfordshire OX26 4LD (GB)**

(72) Inventors:
• **DECOMBEL, Samantha
Bicester, OX26 4LD (GB)**
• **GRICE, Stuart
Bicester, OX26 4LD (GB)**

(74) Representative: **HGF
HGF Limited
1 City Walk
Leeds LS11 9DX (GB)**

Remarks:
This application was filed on 06-10-2023 as a divisional application to the application mentioned under INID code 62.

(54) **DETERMINING AN OPTIMAL WELLNESS REGIME**

(57)   A method of determining an optimum wellness regime is described. The method identifies one or more traits - one or more physiological, behavioural and/or biological states of the user; identifies one or more genetic factors and one or more environmental factors of the user that are relevant to one or more of the traits; assigns a weighting to each genetic and/or environmental factor for the traits that defines the likely effect that the genetic and environmental factor has on the traits; calculates a trait score for each trait based on the weightings assigned to each trait, each trait score indicative of the likely biological, behavioural or physiological response of the user to the trait; defines a trait profile for the user from the trait scores, said trait profile characterizing the users likely biological, behavioural or physiological response to a wellness regime; and determines, based on the trait profile, a wellness regime that is optimised to the goals of the user.

Figure 1

EP 4 280 225 A2

**Description**

Field

The following application relates to a method of determining an optimum wellness regime. In particular a method that identifies one or more traits, said traits being one or more physiological, behavioural or biological states of the user that are potentially relevant to the wellness goals; identifies one or more genetic factors of the user that are relevant to one or more of the traits; identifies one or more environmental factors of the user that are relevant to said one or more of the traits; calculates a trait profile characterizing the user's likely biological, behavioural or physiological response to a wellness regime; and determines a wellness regime that is optimised to the wellness goals of the user is described.

Background

A person's overall health and wellbeing may be the result of a number of different factors. Genetic and environmental factors (which include medical history, fitness activity, nutritional choices, air quality, geographic location and mental health, as well as other non-genetic factors including lifestyle choices, access to fitness equipment, monetary considerations, etc.) can all have an effect on the overall health and wellbeing.

However, even for factors which are known to have positive effects on the health of a majority of individuals, there still exists no mechanism for an individual to understand how these factors relate specifically to them, how they interact with each other, the compounding effect they have on each other, and how they may be utilised in improving that person's overall health and wellbeing.

Thus, it is highly desirable to develop a system in which a user submits environmental factors/data (potentially including lifestyle data/parameters) and genetic factors/data, specifies health-related goals, and receives a visually-driven diet and fitness recommendation package, known as a wellness regime, that provides guidance regarding diet, nutrition, exercise and sleep, detailing how their lifestyle decisions and wellbeing regime need to be altered to meet those goals more effectively, taking into account the impact of these genetic and environmental factors on the user.

Most health and fitness platforms that utilise genetics are too generic and give advice that does not take into account the complex compound effects of interactions between different genes and different environmental factors - they are only based on the genetic and/or environmental data specific to one biological facet in isolation. The diet / exercise recommendation outputs they produce are often not the most optimal for the user and can even cause detrimental effects by giving inadequate and ill-catered fitness and nutritional advice given a specific individual's particular environment/ lifestyle.

The prior art does not provide a system which uses genetic and environmental data to define a number of "traits", or propensities towards particular phenotypic-expressions and then use these traits to model a smaller number of higher-level biological systems, which can give more accurate and personalised health and fitness advice; there is no intermediate step. Nothing in the prior art discloses calculating and using the compounding effects of trait to trait interactions on a biological system level, which means dietary and fitness advice delivered by the systems in the prior art could be incorrect, and even detrimental to a user, because the compound interactions of traits and biological systems (i.e. the interaction between genetic and environmental factors) are not taken into account.

Within the prior art, many systems make recommendations based on a certain characteristic of a user - for example, a particular facet of their environmental lifestyle choices or a subset of genetic data.

*Using testosterone levels* as *an example to show the deficiencies of the prior art.*

Using the methods discussed in the prior art, if a user performs a medical or genetic test which reveals they have low testosterone levels based on diet choices and fitness activity, or that genetically they are susceptible to have lower testosterone levels, many of the methods discussed within the art will automatically recommend the user to partake in diet, fitness and lifestyle programs that are likely to raise their testosterone levels. The art does not take into account the compound effects of having low testosterone levels on the other biological systems within the body. Further, it also does not consider the effect that recommending programs to raise these levels may have on the other biological systems in the body. Although the methods disclosed in the art may assess a user on a biological system by biological system or trait by trait basis, they do not provide a more comprehensive biological trait profile model of the user where the compound effects of environmental, lifestyle parameters and the genetic data of the individual are taken into account, providing an optimally balanced and personalised view to achieving the user's goals.

This may mean, for example, that the advice and recommendations offered to the user may temporarily raise total testosterone levels (such as increased fat intake) or free testosterone levels (such as increased glucose intake) within their body, although the compound and resulting effects of this recommendation could have detrimental effects on other traits (such as insulin sensitivity or blood lipid level) and ultimately systems within their body and cause subsequent adverse effects on the user's health, which have an overall negative sum impact - such as increasing body fat. Without a complete understanding of the physiological makeup of the biological systems within the body, it may not be possible to give reliable diet and fitness advice to the user that is best tailored towards their profile.

Summary

According to a first aspect of the present invention, there is provided a method of determining an optimum wellness regime that best meets the wellness goals of a user, said method comprising the steps of: identifying one or more traits, said traits being one or more physiological, behavioural and/or biological states of the user; identifying one or more genetic factors of the user that are relevant to one or more of the traits;
identifying one or more environmental factors of the user that are relevant to said one or more of the traits; assigning a weighting to each genetic and/or environmental factor for the traits, said weighting defining the likely effect that the genetic and environmental factor has on the traits; calculating a trait score for each trait based on the weightings assigned to each trait, each trait score indicative of the likely biological, behavioural or physiological response of the user to the trait; defining a trait profile for the user from the trait scores, said trait profile characterizing the users likely biological, behavioural or physiological response to a wellness regime; determining, based on the trait profile, a wellness regime that is optimised to the goals of the user.

The present invention provides a mechanism for correlating genetic and environmental factors to provide a wellness regime that is optimised for the user. The present method determines or identifies a number of traits that are potentially relevant to the goal of the user, such as testosterone level, VO2 max and power ability for a user intending to increase their muscle mass. Genetic and environmental factors relevant to the identified traits are then identified. These factors may be identified from a predetermined listing, or the factors may be determined based on feedback from the user, or based on collated information of an ensemble of users. For example, by analysing users having similar environmental factors, genetic factors influencing traits may be determined. As a specific example, for testosterone genes, genetic variations associated with the ESR1, ACTN3, SHBG1 and SHBG2 are considered relevant genetic factors, whilst body type, body fat %, age, activity levels, weekly active hours and sex may be relevant environmental factors. From these factors, a trait score may be determined. Weightings can be used to provide a statistical weighting to the factors - it can be appreciated that age, sex and activity levels may provide a greater weighting to overall testosterone levels than one or more of the genes, and visa-versa.

The trait score is intended to be indicative or define the likely biological, behavioural or physiological response of the user to the trait. Additionally, or alternatively, an external test or result may be used. For the earlier testosterone example a blood testosterone reading may be used to determine a precise trait score.

From the trait scores, a trait profile may be defined for the user. Such a profile acts as an individual indicator of a user's likely biological, behavioural or physiological response to variations in genetic, environmental or behavioural factors and accordingly can be used to characterize a user's response to a wellness regime. From this information, a wellness regime that is optimised to the wellness goals of the user can be determined.

In an embodiment, one or more of the traits may comprise a plurality of trait bands, each trait band being indicative of particular trait outcome. Furthermore, the step of calculating a trait profile may further comprise the step of: assigning a trait score of one or more of the traits to one of the trait bands, depending on the relative strength and directionality of the trait score. By directionality, it is intended to describe whether the trait score is positive or negative, which may correlate to a very high or very low banding suggesting a very high or very low effect of the selected trait within the user's trait profile. Traits can also be unidirectional, or may be defined by any other distribution type.

It can be appreciated that the trait bands may be a grouping of outcomes that describe the user's expected or natural response to the trait being considered. For example, for the trait testosterone 5 bands may be provided, ranging from very low to very high, where each band is indicative of predicted current and/or future testosterone levels at current activity levels and/or at expected activity levels if the user implements the wellness regime optimised to their goals suggested by the method.

The step of determining a wellness regime may further comprise the step of: determining one or more recommendations, said recommendations providing an actionable outcome for the user. The recommendations may be determined based on one or more of: trait scores; and/or trait profiles; and/or data elements, said data elements comprising one or more genetic factors, and/or one or more environmental factors and/or one or more wellness goals.

Data elements are intended to provide extra resolution to the recommendations, but are not classed as traits in themselves e.g. one example is a that a recommendation of the Polyunsaturated fat ratio in a diet is determined by the trait defining omega 6 intake (or omega 3 to omega 6 fat ratio) plus a data element describing diet, which means if someone is recommended to increase their omega 3 intake, then a sensible recommendation on how to do this is also based on their diet i.e. if they are vegetarian, a recommendation isn't made to eat more fish.

As an additional example, a recommendation may be provided to the user regarding the total amount of protein that should make up the user's diet. This may be provided as a % of food intake, kcal intake, or grams. The recommendation may be given to the user in bands, ranging from very low to very high. The recommendation level or band selected in this instance depends on the trait scores for the traits describing satiety, insulin sensitivity, obesity risk and lean body mass. Non-trait data elements that are relevant to determining the banding or level of recommendation for this example include age of the user, intermediate/active/ultimate goals of the user and goals of the selected wellness regime.

Like with the trait scores and banding, the recommendations may be weighted depending upon the trait scores or trait bands or data elements which influence the recommendation. For example, a recommendation on whether to supplement the users diet with vitamin B12 that is dependent on the trait describing folate metabolism and the environmental factor diet is likely to be heavily weighted to diet. It can be appreciated that other weightings may be appropriate for other recommendation.

Weightings and/or recommendations may also be generated based on user data, either from feedback from the present user or from a database having storage of all users response either from surveys or further physical data, that can then be fed back to improve the accuracy of the method.

The traits may include, but are not limited to, one or more of insulin sensitivity; obesity risk; gut microbiome profile; blood testosterone levels; dyslipidaemia, lactose intolerance, resting blood triglycerides level, resting blood glucose levels, oxidative muscle dominance, saturated fat level, satiety, folate metabolism and conversion, homocysteine levels, methionine requirement, caffeine metabolism, drug metabolism, susceptibility to injury, hypertension levels, circadian rhythm, sleep disturbance, sleep cycles, emotional stress levels, physical stress levels, susceptibility to stress, trainable VO2 max, salt sensitivity, workout recovery between workout sessions, workout recovery during a workout session, lactate clearance levels, basal metabolism, lean body mass, endurance capability, power capability, conscious restraint, binge eating propensity, emotional eating propensity, eating behaviour, resting LDL and HDL levels, resting metabolite levels, macronutrient requirements, micronutrient requirements, lean muscle mass %, body fat %, fat usage and metabolism, carbohydrate usage and metabolism, response to omega 6, response to diets types, response to omega 6, biological age, and susceptibility to aging. It can be envisaged that other traits may be identified, particularly traits that are relevant to a wellness regime and dependent on a user's genetic and/or environmental condition.

The likely physiological, behavioural and/or biological response of the user to a wellness regime indicated by the trait scores, in particular the likely response that the genetic factor has on the traits and/or the likely response that the environmental factor has on the traits, may be an expected physiological/behavioural/biological/genetic or environmental response of a typical user based on comparisons between the user and an existing known user having identical or similar genetic factors, or identical or similar environmental factors or an identical or similar wellness regime. Additionally, or alternatively, the likely responses may be predicted responses based on empirical data and/or statistical analysis of prior users. It can also be appreciated that the weightings applied to the genetic factors and/or the environmental factors may be determined in a similar manner using expected or predicted data or statistical analysis.

The wellness regime is typically a health, and/or nutritional and/or fitness regime. In particular, the wellness regime may be one or a combination of a nutrition plan, wellbeing plan, fat loss plan, muscle building plan, strength training plan, microbiotic supplementation plan, or a medical regime.

In examples, the weighting of a trait can be zero, positive or negative. It can be appreciated that negative values may be used that indicate a contra-indication.

The genetic factors may be genetic variants. The genetic variants may include polymorphisms and gene copy number variants.

In embodiments, the trait profile may characterize the user's biological system. The trait profile may be considered to be the relative levels or propensity to predefined levels or factors.

At least one genetic factor, and/or at least one environmental factor, may be relevant to multiple traits.

The weighting assigned to the genetic factors, and/or the weighting assigned to the environmental factors, may be different for different traits.

A trait score may be calculated from the interaction between one or more genetic factors, and/or between one or more environmental factors, and/or between a combination of genetic and environmental factors, for each or a combination of traits. For example, trait scores for the traits of low satiety and lactose intolerance may be determined or calculated based on a single genetic factor, although it can be appreciated that additional relevant genetic and/or environmental factors relevant to the traits may be added as applicable. The interaction may be one or more of hierarchical, synergistic, additive, subtractive or antagonistic.

A trait score may also be modified by behavioural measures, personality characteristics and/or psychometric analysis, such as the Three-Factor Eating Questionnaire.

A trait score may be modified by direct biophysical data. The direct biophysical data may be data defining the actual biological or physiological response of the user to one or more of said parameters. The direct biophysical data may be provided by a data interface, by a third party biophysical data system or by the user.

The trait profile may be calculated from the hierarchical, synergistic, additive, subtractive or antagonistic interaction of the trait scores.

A separate trait score may be calculated for each expected physiological or biological response of the user to each separate trait.

The trait profile may characterise the user's biological system. For example, a trait may be considered the user's propensities to a particular physiological or biological read-out or variable. Typical physiological or biological read-outs can include one or more of: insulin sensitivity; blood testosterone levels; dyslipidaemia and lactose intolerance, although many others can be envisaged.

The step of determining/calculating a trait score may comprise the step of: cross-referencing the one or more genetic factors and/or the one or more environmental factors relevant to said at least one trait and calculating a trait score based on the cross-referencing. This trait score calculation can consider the relative genetic-genetic, genetic-environmental and environmental-environmental interactions. These cross-referencing interactions can take many forms, and may be hierarchical, synergistic, additive, subtractive or antagonistic, for example. Each trait score calculation is typically particular to that said trait and considers known physiological, behavioural or biological variables that impact on that trait.

Traits scores may then be used to calculate a biological system level profile for a user (the biological system models). The step of calculating a biological system model may comprise the step of weighting the one or more traits according to the impact those physiological or biological variables have on each specified biological system (the trait profile for that particular biological system). This results in a specific read-out for each biological system modelled, which, either in combination or individually, can be used to predict an individual's propensity to respond to a particular regime or intervention. Each biological system model read-out may be generated from a single or a number of trait scores. When the biological system model is calculated using the trait profile, the analytical methods for cross-referencing between the trait scores and profiles can take many forms, and may be hierarchical, synergistic, additive, subtractive or antagonistic, for example. It can also be appreciated that the user's biological system model may be calculated from or comprise a plurality of trait profiles.

Trait scores may be used to provide a relative indication of a trait's propensity to affect a particular physiological or biological read-out, or variable. Accordingly, the trait profile at the biological system level can also be considered to be a relative indication of a user's overall physiology or biology, and ultimately their propensity to respond to a particular regime.

A trait profile may be calculated by weighting the trait scores according to the wellness goals of the user.

In examples, the weighting for trait score of a trait in a trait profile for a particular biological system can be zero. This means that a selected trait may provide no indication of a user's propensity to a particular variable or regime, or that any effect of the trait for that biological system does not differ from the default or reference position. It can be appreciated that negative values may be used that indicate a contra-indication.

In embodiments, traits may initially have a default trait score indicating the average predicted response of a defined average user to the trait. The default trait score can then be updated with the trait score for a specific user. It can be appreciated that the average user may be selected or determined to match the gender, activity level, age, race etc. of the user. An average user may be a male, aged 25 to 40, having moderate exercise and activity levels and a moderate diet. It can be appreciated that the average user may be a construct used for the statistical model.

By utilising such default scores, in the case of missing or incomplete genetic and/or environmental factors, an estimated trait score can be used to ensure that a regime that requires a value for a trait score can be included in the determination step or process.

The biological system models may be used to characterise the user's overall physiology or biology. In examples, typical biological systems can include one or more of: metabolism, skeletal muscle system, body fat, liver function, oxygen transport, circulatory system, central nervous system, metabolome, epigenome and gut environment, although many others can be envisaged.

The biological system models may be considered to be a representation of an individual's physiological and biological propensities and health status. Such models may then be used to help predict/identify how an individual will respond to different health, nutritional and/or fitness programs, and, importantly, to match them to the optimal system for their goals and/or needs.

In examples, different health, nutritional and/or fitness programs may be determined for different biological system models. Typical queries relating to health, nutritional and/or fitness programs may include: 'How many calories should I consume?', 'What type of exercise should I do?', 'How frequently should I train?', 'What proportion of protein should I eat?', although many others can be envisaged.

In examples, different wellness regimes (health, nutritional and/or fitness programs) can be matched to users goals or needs. Typical goals or needs may include fat loss, muscle building, endurance training, cardiovascular improvement, rehabilitation from illness/disease, disease prevention, although many others can be envisaged.

In embodiments, the step of determining a wellness regime may comprise the steps of matching said trait scores to one or more default, or reference traits. The reference traits may be associated with reference trait profiles, said reference traits having reference trait scores defining the response of a reference group of users to a physiological or biological variable. Accordingly, the reference trait profiles may provide an overall reference response of a particular group of users. By utilising reference traits and reference trait profiles for large groups of users, statistical analysis and learning (machine or otherwise) can be used to match or predict the likely response of the user based on the difference between their traits and trait profile and the reference traits and trait profile. Artificial intelligence, such as IBM Watson (RTM) may be employed to predict the likely response of the user and/or to predict an optimal wellness regime. As the reference trait database grows, the accuracy of the matching between the predicted response and the actual user response should improve.

In embodiments, the genetic and environmental factors may be scored according to ensemble research to determine a genetic or environmental factor score assigned weighting/value indicative of the user's genetic or environmental factor compared to the ensemble research. The trait scores may also be determined or modified using the factor score of each one or more genetic and environmental factor.

In another example, the step of determining a wellness regime may include the steps of: identifying a wellness (health, or performance) goal of the user; querying a database of traits relevant to the said goal; and prioritising the traits identified by the database for the said goal. The prioritising can comprise the steps of: adjusting the trait scores of the user corresponding to the traits identified by the database according to weighting factors stored within the database; and updating the trait profile based on the adjusted trait scores.

Utilising goals allows the method or system to tailor a health, nutritional and/or fitness regime to match the goals and/ or needs of the user. It can be appreciated that many users have a goal or ambition that they wish to achieve from a health, fitness or nutritional regime. It may be appreciated that the optimum regime for the user's goal may not be the same at any point in time, if the user's goal is incompatible or unsuited (perhaps temporarily) with the user's trait profile. For example, a pre-existing health condition or situation may need to be addressed first to enable the ultimate goal to be achieved more effectively and/or safely. In such a scenario, the method or system can select a regime that it considers to be in the best interests of the user. This may be considered to be an active goal for the user, which may differ from the user's ultimate goal. For example, the ultimate goal of the user may require a base level of strength or physical fitness to achieve, therefore the active goal determined by the method or system instead provides a plan for user to first reach the prerequisite base levels for the ultimate goal to be achieved.

In one embodiment, the step of matching the user with a known wellness (health, nutritional and/or fitness) regime comprises the steps of: cross-referencing the user's trait profile with reference trait profiles associated with a databank of known regimes; and determining one or more optimum regimes from the databank of regimes, by selecting one or more known regimes deemed most beneficial by relatedness of user trait profile to reference trait profiles. The association of reference trait profiles with particular health, nutritional and/or fitness regimes may be calculated by reference to academic studies and published literature. As noted above, this data can be modified using artificial intelligence to monitor user feedback and refine the matching process.

It can be appreciated that having the user provide a feedback response regarding the health, nutritional and/or fitness regime allows the databank of trait profiles matched to health, nutritional and/or fitness regimes to be improved for future users. Additionally, based on the feedback response, the trait scores, trait profiles or, where applicable, the factor weightings can be altered and the system or method reanalysed to update the health, nutritional and/or fitness regime suggestion. The feedback response may be collated within the system or a databank, which may be a sandbox environment. The feedback response may result in one or more updated environmental or genetic factors. The trait score of the one or more traits may be altered based on the feedback.

In examples, feedback response can be measured using questionnaire, app technology, wearables, lab based studies and clinical trial, although many others can be envisaged.

In response to feedback the trait score may be altered within a sandbox database until the overall predicted response of the user matches the feedback response, to test the overall higher impact of the change. This allows the system or method to adapt and analyse and update the associated scores and rules without potentially negatively affecting the user. Once the sandbox analysis is complete and verified, the updated trait values may be applied to the traits of the user within a live database.

The genetic factors, environmental factors and traits may define a profile for the user. The genetic factors may be identified from one or more of: a laboratory DNA test; a user DNA test; or medical laboratory data. The environmental factors may be identified, for example, from one or more of: health and fitness applications; questionnaires; mobile applications; laboratory data; health and fitness monitors or a computing device.

The step of determining which wellness regime is optimised may include the steps of: identifying a wellness goal of the user; querying a database of traits relevant to wellness goals for the wellness goal; and prioritising the traits identified by the database for the wellness goal. In such embodiments, the step of prioritising may comprise the steps of: adjusting the trait scores of the user corresponding to the traits identified by the database according to weighting factors stored within the database; and updating the trait profile based on the adjusted trait scores.

The step of determining which wellness regime may also comprise the steps of: cross-referencing the user's trait profile with trait profiles of other users of a databank of known wellness regimes; and determining one or more optimum wellness regimes from the databank of wellness regimes, by selecting one or more known wellness regimes deemed most beneficial by users of similar trait profiles.

The step of determining the databank of wellness regimes may be from a third party databank of external wellness regimes.

In embodiments, the user may provide a feedback response regarding the health regime. Furthermore, the step of predicting the optimum wellness regime may comprise the step of selecting a wellness routine based on previous user feedback responses. The user feedback may be provided by an interfaced system, optionally a third party system, although it can be appreciated that a complimentary system provided by the provider of the method may be used. The feedback response may be collated within the system or a databank of known health regimes. Additionally or alternatively, the feedback response may be used to update the weightings of one or more of the environmental factors (or conceivably the genetic factor weighting). The trait score of the one or more traits may also be altered based on the feedback.

Additional genetic and/or environmental factors may also be later determined for certain traits. As necessary, such additional factors may be used to update the weighting of the factors (and also therefore the trait scores and trait profile).

The trait score may be altered within a sandbox database until the overall predicted response of the user matches the feedback response. The trait values may then be supplied to the trait profile within a live database.

The genetic factors, environmental factors and traits may define a profile for the user.

The genetic factors may be identified from one or more of: a laboratory DNA test; a user DNA test; or medical laboratory data. Such data may be provided from third party companies via data downloads, API's or other known data transfer mechanisms.

The environmental factors may be identified from one or more of: health and fitness applications; questionnaires; mobile applications; laboratory data; health and fitness monitors, user location, user psychological history, location conditions, or a computing device. A user's medical history may also be considered.

The wellness regime may be predicted using an artificial intelligence computing device.

The method may comprise the step of exporting information representative to said trait profile to a third party server or external server. Such information may be considered to be a digital fingerprint representation of the trait profile, or the trait profile itself. The digital fingerprint may be used to ensure an additional level of security for the trait profile. The digital fingerprint may restrict access to the trait scores and the other raw data such as the weightings, for example, restricting third party access to the raw data, whilst allowing interfacing with third parties via an API (application programming interface). The method may further comprise the step of providing advertising to the user tailored to the trait profile.

The method may further comprise the step of interfacing with external data sources to supplement and/or incorporate user data within the trait profile. The interface may be via a mobile application.

The external data sources may interface with a server implementing the method such that the server provides a guided manual for analysis of the external data. Accordingly, such an example may provide a black-box arrangement where data is provided to the server from external data sources, with wellness regimes and/or other requested data provided by the server. In this manner, the server may be used to provide restricted access to one or more trait or environmental or other user specific data to allow additional applications to be run based on the data. It can be envisaged that such other apps may utilise for example the caffeine metabolism trait score to determine which coffee bean to advertise/ recommend to an individual, for example.

It can be envisaged that such other apps may utilise for example the insulin sensitivity trait score, in addition to other factors, to determine a patient's risk of developing diabetes, for example.

According to a second aspect of the invention there is provided a system for carrying out the method described in the first aspect.

As broadly defined herein, aspects of the invention may define a method having at least some of the following steps: identifying one or more genetic variants (including, but not limited to, polymorphisms and/or insertions and/or deletions and/or gene copy number variants) of a user, with each of these genetic factors being assigned a weighting/value that is used to calculate the likely, typically the expected or a predicted, response on a physiological, behavioural or biological parameter, called a 'trait'. Each genetic factor can impact one or multiple traits. The weightings assigned to the genetic factors can also be different for different traits. This may allow the weighting assigned to each genetic factor to be different when applied to different traits.

Identifying one or more environmental factors of a user, with each environmental factor being assigned a weighting/value that is used to calculate the likely, typically the expected or a predicted, response on a trait can then be performed. Each environmental factor can impact one or multiple traits. The weightings assigned to the environmental factors can also be different for different traits. This may also allow the weighting assigned to each environmental factor to be different when applied to different traits.

Calculating a score for a 'trait', which is a defined physiological, behavioural or biological read-out, using the combined genetic and environmental weightings/values that have been scored for that trait, to generate a trait score can then be performed. The trait score calculation may consider the relative genetic-genetic, genetic-environmental and environmental-environmental interactions. These interactions may be hierarchical, synergistic, additive, subtractive or antagonistic, for example. Additionally, a predicted trait score may be modified by direct biophysical data, where known.

Trait profiles may then be used to calculate models of an individual's biology systems and ultimately an individual's health and fitness status (the 'biological system' level). Each biological system model may be generated from a number of trait scores (the trait profile for that biological system). When a biological system model is calculated using the trait profile, the interaction between different trait scores may be hierarchical, synergistic, additive, subtractive or antagonistic. The biological system models can be used, either in combination or individually, to help, or to solely, predict how an individual will respond to different health, nutritional and/or fitness programs, and to match them to the optimal system for their goals and/or needs.

This approach considers the collective effects of both genetic and environmental factors in determining health, nutritional and/or fitness regimes for a user. By utilising both factors in combination, the ensuing biological system models, which are predicted by the trait profile, may be used to calculate a more personalised and accurate biological and health profile of the user. An additional key part of this method is that trait scores can also be used in combination to infer a higher level of understanding of an individual, and ultimately allow for more targeted and accurate recommendations. This ensures that a broader perspective and consideration is given to predicting responses to: lifestyle choices such as diet and exercise; identifying health and medical risks, and prioritising health, nutritional and/or fitness regimes. The regime may be a health, diet, and/or fitness plan; or more generally, an indication of how to improve or maintain or prevent decline of a user's physical or mental condition; or specific, medically tailored recommendations.

The present disclosure describes providing a system and method which uses a combination of user-specific relevant genetic, environmental and lifestyle data to define a number of "traits", which is a defined physiological, behavioural or biological read-out, and then using these traits to model a smaller number of higher-level biological systems. When the overall system or method is queried as to what health regime, fitness regime or diet would meet a particular desired goal, an algorithm or artificial intelligence system may be used to match wellness (medical/lifestyle/fitness/training/diet) advice with the user's specifically defined biological system and trait profile, including the compounding effects of trait-trait interactions at the biological system level.

The embodiments described herein relate to collecting and analysing user-specific genetic and environmental data to develop comprehensive, personalised diet and fitness programs for helping a user achieve their desired goal or a better health status. The user-specific data may be collected from a variety of sources, including, but not limited to, genetic testing, lab testing, nutrition information, physiological tests, health tests, metabolic testing, psychological testing, mobile health devices worn by the user and applications through which the user manually inputs information.

The user-specific data is collected and analysed together, based on knowledge of the interrelationships between general genetic and environmental data, to develop a user profile with personalised diet and fitness programs that are targeted to improving specific areas of the user's health by implementing changes in exercise, nutrition, environment etc. These plans are highly personalised as they are based upon a person's own genetic code and their environmental condition / lifestyle choices.

A user interested in finding a health, diet or fitness plan, which is optimal for their own personal genetic make-up and their health and lifestyle history, and one that may be targeted at achieving a specific goal, may submit a genetic sample, for example saliva, for genetic information extraction. The received sample containing genetic material may be processed to produce a genetic readout suitable for analysis at a databank. Stored algorithms having parameters which depend upon genetic features may be applied to the data to produce an output to characterise a user in terms of their genetic factor score.

Further, the system uses the genetic and environmental data to define a number of "traits", or propensities towards particular phenotypic expressions, and then uses these traits to model a smaller number of higher-level biological systems. A trait, as defined here, may be a physiological, behavioural or biological metric based on genetic and/or environmental factors; for example, insulin sensitivity; obesity risk; gut microbiome profile; blood testosterone levels; dyslipidaemia, lactose intolerance, resting blood triglycerides level, resting blood glucose levels, oxidative muscle dominance, saturated fat level, satiety, folate metabolism and conversion, homocysteine levels, methionine requirement, caffeine metabolism, drug metabolism, susceptibility to injury, hypertension levels, circadian rhythm, sleep disturbance, sleep cycles, emotional stress levels, physical stress levels, susceptibility to stress, trainable VO2 max, salt sensitivity, workout recovery between workout sessions, workout recovery during a workout session, lactate clearance levels, basal metabolism, lean body mass, endurance capability, power capability, conscious restraint, binge eating propensity, emotional eating propensity, eating behaviour, resting LDL and HDL levels, resting metabolite levels, macronutrient requirements, micronutrient requirements, lean muscle mass %, body fat %, fat usage and metabolism, carbohydrate usage and metabolism, response to omega 6, response to diets types, response to omega 6, biological age, and susceptibility to aging.

This approach considers the effect of both genetic and environmental (which includes lifestyle) factors in determining a health regime for a user. By utilising both factors, the trait profile may provide a customised profile of the user and their likely response to variables such as diet, exercise, medication or the like. The trait profile can consider the relative interactions between genetic-genetic factors, genetic-environmental factors and environmental-environmental factors. This ensures that a broader perspective and consideration is given to selecting a health regime that takes account of both genetic and environmental relevant factors, rather than purely focussing on, typically, genetic issues as has been generally the case. The wellness regime may be a health and/or fitness plan or regime or more generally an indication of how to improve or maintain or prevent decline of a user's physical or mental condition. The wellness regime may further be one or more of nutrition plans, wellbeing plans, fat loss plans, muscle building plans, strength training plans, endurance training plans, sporting performance plans, microbiotic supplementation plans, medical regimes or any other plans designed to target a specific aspect of the user's wellbeing.

Additionally, aspects of the present invention may include a compliance factor, wherein the method further comprises one or monitoring sensors or devices that are configured to analyse a user's compliance with the determined wellness regime. Based on the information collated by the sensors or devices, the wellness regime may be updated or adjusted in real time to either ensure that the original goals of the user are met or alternatively to provide an alternative intermediate or active goal for the user to aim towards. This may be useful for the user to allow them to understand or appreciate how deviating from the suggested recommended wellness regime influences their chance of achieving their wellness goals.

There may be provided a computer program, which when run on a computer, causes the computer to configure any apparatus, including a circuit, controller, sensor, filter, or device disclosed herein or perform any method disclosed herein. The computer program may be a software implementation, and the computer may be considered as any appropriate hardware, including an analyser, a microprocessor, and an implementation in read only memory (ROM), erasable programmable read only memory (EPROM) or electronically erasable programmable read only memory (EEPROM), as non-limiting examples. The software implementation may be an assembly program. A user interface may be provided to allow for the inputting of information by the user - such interface may be a webpage, application or hardware based interface.

The computer program may be provided on a computer readable medium, which may be a physical computer readable medium, such as a disc or a memory device, or may be embodied as a transient signal. Such a transient signal may be a network download, including an internet download.

It can be appreciated that, although certain examples and embodiments described above have been primarily described with respect to a single aspect, the features described are also applicable to the other aspects defined herein.

These and other aspects of the invention will be apparent from, and elucidated with reference to, the embodiments described hereinafter. The above discussion is not intended to represent every example embodiment or every implementation within the scope of the current or future Claim sets. The Figures and Detailed Description that follow also exemplify various example embodiments. Various example embodiments may be more completely understood in consideration of the following Detailed Description in connection with the accompanying Drawings.

Brief description of Drawings

Embodiments will be described, by way of example only, with reference to the drawings, in which

Figure 1 shows a high-level system diagram of the disclosed invention, identifying the primary stages and directional throughput.

Figure 2 shows the Genetic and Environmental Data Collection environment and some of the collection mechanisms that may be used within an embodiment of the system shown in Figure 1.

Figure 3 shows an embodiment compatible with figures 1 and 2 of a mechanism for calculating the trait scores for a specific user based on the genetic and environmental data collected.

Figures 4a-c shows a preferred detailed embodiment for a Trait and Biological System Model described in Figures 1 to 3.

Figure 5 shows an embodiment of the system of Figure 1 where bandings and recommendations are shown.

Figure 6 shows an example of a user interface according to an embodiment of the disclosed invention.

It should be noted that the Figures are diagrammatic representations and not drawn to scale. Relative dimensions and proportions of parts of these Figures have been shown exaggerated or reduced in size, for the sake of clarity and convenience in the drawings. The same reference signs are generally used to refer to corresponding or similar features in modified and different embodiments.

Detailed description of embodiments

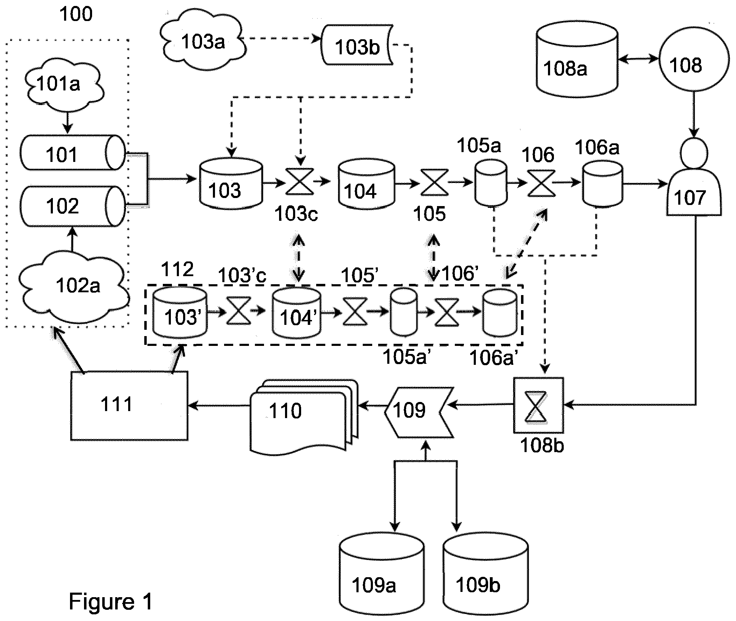

Figure 1 shows a high-level system diagram of the disclosed invention, identifying the primary stages and directional throughput. Dotted lines are used to represent review stages that are updated as the dataset increases.

The following labels within Figure 1 are defined as:

- **101** - **Genetic Data** - Digital information regarding the genetic make-up of a user which defines the genetic potential of a user carried in the base sequence of their DNA according to the genetic code.
- **101a** - **Genetic Data collection** - DNA collection which includes, but is not limited to, a saliva sample home collection kit and instructions.
- **102** - **Environmental Data** - Digital information regarding the health, lifestyle and wellbeing of the individual which may include, but is not limited to, height, weight, age, blood pressure, body fat percentage and fitness activity.
- **102a** - **Environmental Data collection** - Multiple methods of inputting environmental data into the system which may include, but are not limited to, health monitoring applications or devices, questionnaires and professional health data.
- **103** - **Databank** - a database that contains genetic and environmental data which is linked with the demographic data of an individual prior to analysis and characterisation.
- **103a** - **Industry Knowledge** - Academia and knowledge used to characterise the user based on analysis of their genetic and environmental data.
- **103b** - **Rules/Tags** - Rules and tags extracted from the source/industry knowledge that is used to characterise the databank.
- **103c** - **Algorithm** - An algorithm to tag and categorise the environmental and genetic data, using the defined rules/tags, 103b, to further define and categorise a user.
- **104** - **Refined Databank** - A database containing further defined and categorised genetic and environmental information about a user based on the data collection, analysis and calculation performed on the initial data within 103. The refined databank contains highly user-specific genetic and environmental scores.
- **105** - **Trait Scoring Algorithm** - An algorithm used to produce a physiological or biological readout, a "trait score", for the individual based on combinations of the environmental and genetic scores of the user.
- **105a** - **Trait Scores** - A quantitative physiological or biological readout for one or more traits of a user based on genetic and environmental data, for example, resting blood triglycerides level.
- **106** - **Evaluation** - Means of characterising biological systems of a user based on the trait scores 105a.
- **106a** - **Biological System Characterisation** - A readout for a physiological understanding of defined biological systems of a user, based on a combination of traits that represent the trait profile for that biological system.
- **107** - **User profile** - Detailed, annotated understanding and characterisation of the user which is created by utilising customised algorithms 105 106 and the data within the refined databank 104 which leverage understandings of the relationships, the interactions and the compound effects between the genetic, fitness and environmental data.
- **108** - **Goal Linked Questions** - A health, fitness and/or diet goal for a user such as "increase endurance", "build muscle" or "lose fat".
- **108a** - **Question and Search Methods** - A database of fitness goals and diet goals, which are tagged with the necessary search information to find, order and prioritise relevant traits within the biological systems to give the appropriate diet/fitness recommendation's
- **108b** - **Goal-specific Trait and Biological System Model** - An algorithm used to modify scores based on weightings of traits associated with particular goal-linked questions and their effects on the biological systems to produce a readout.
- **109** - **Fetch Plans** - User-specific fitness and diet plans are fetched from the databanks and are based on the outputs of the trait and biological system model.
- **109a** - **Databank of Fitness Plans** - Fitness plans categorised based on industry knowledge, academia and other sources.
- **109b** - **Databank of Diet Plans** - Diet plans categorised based on industry knowledge, academia and other sources.
- **109c Databank of Wellness Plans** - Wellness plans categorised based on industry knowledge, academia and other sources.
- **110** - **User Interface for delivery of plans to User** - user interface to deliver diet, fitness and wellness plans as well as translated information on user profile to the user via a graphical user interface.
- **111** - **User-reported feedback and monitoring** -information on user progress is returned via online, mobile or third party surveys, applications or devices.
- **112** - **Feedback Analysis** - a sandbox environment uses user-reported data to continually assess and refine the model, test the calculations and evaluate outputs using updated and real-time data.

Figure 1 illustrates an embodiment of the system for collecting and analysing user-specific data 100 to develop comprehensive personalised diet and fitness programs. In this embodiment, user-specific genetic and environmental data 100 is collected from multiple sources 101a and 102a such as:

- Genetic Tests
- Mobile health monitoring applications
- Questionnaires

User data, also called factors, 100 is collected and stored in the central databank 103, the databank 103 generally contains genetic 101 and environmental 102 factors/information for a specific user. But within the databank at this stage, there may be no weightings or defined characterisation of the user. The received genetic data/factors and environmental data/factors, 101 102 is collected within the databank which stores all relevant information on the user to be defined in the model. The genetic data 101 is assessed to give a very high level overview of the user, for example, "user x has gene y and specific variant z in regards to gene y". Further, the information within databank 103 is used to identify the presence or absence of certain specific genetic features or markers related to diet, fitness and health.

Further analysis then takes place based on the interrelationships between genetic and environmental data to characterise the user based on the interrelationships and compound effects defined by a combination of the rules/tags 103b, the industry knowledge 103a and the user-specific data 100 within the databank 103.

103a "industry knowledge" can include information from academia, health care professionals, research scientists, health and fitness applications etc. and is used to create rules/tags 103b. These rules/tags are used to categorise and sort the genetic and environmental data in the databank 103 into the refined databank 104.

In one embodiment, after analysis and computing of a user's genetic and environmental data 100 in combination with the industry knowledge 103a and rules/tags 103b, a more defined understanding and characterisation of the user will be present and is stored within the refined databank 104.

In this embodiment, the refined databank 104 typically contains further defined and categorised genetic and environmental information about a user based on the data collection, analysis and calculations performed 103c. The refined databank 104 can contain highly user-specific scores and weightings, based on genetic makeup and environmental data.

Once the user has been characterised within the refined data bank 104, a trait scoring algorithm 105 uses the data in order to analyse, define and generate a more specific user profile or "avatar" 107.

In particular, trait scores 105a, which define the likely biological, behavioural or physiological response of the user to traits can be calculated. Such traits include but are not limited to, one or more of insulin sensitivity; obesity risk; gut microbiome profile; blood testosterone levels; dyslipidaemia, lactose intolerance, resting blood triglycerides level, resting blood glucose levels, oxidative muscle dominance, saturated fat level, satiety, folate metabolism and conversion, homocysteine levels, methionine requirement, caffeine metabolism, drug metabolism, susceptibility to injury, hypertension levels, circadian rhythm, sleep disturbance, sleep cycles, emotional stress levels, physical stress levels, susceptibility to stress, trainable VO2 max, salt sensitivity, workout recovery between workout sessions, workout recovery during a workout session, lactate clearance levels, basal metabolism, lean body mass, endurance capability, power capability, conscious restraint, binge eating propensity, emotional eating propensity, eating behaviour, resting LDL and HDL levels, resting metabolite levels, macronutrient requirements, micronutrient requirements, lean muscle mass %, body fat %, fat usage and metabolism, carbohydrate usage and metabolism, response to omega 6, response to diets types, response to omega 6, biological age, and susceptibility to aging. It can be envisaged that other traits may be identified, particularly traits that are relevant to a wellness regime and dependent on a user's genetic and/or environmental condition..

The user data 100, in particular the trait scores 105a are analysed 106 using customised algorithms to generate trait profiles 106a which leverage understandings of the relationships, the interactions and the compound effects between the genetic and environmental data in order to generate a user profile 107. This is further explained within the trait scoring section below.

Next, the user profile 107 may be queried with goal related questions 108. In one embodiment, a goal of the user could be, but is not limited to, 'building muscle', 'losing fat', 'improving endurance', 'improving strength' and 'improving cardio-vascular capability' etc. The relevant traits, biological systems and their weightings and hierarchies are specific to the goal, and subsequently the goal related question 108, of the user. For all goal related wellness, fitness and diet based questions, the questions can be tagged with the necessary search information to find, order and prioritise relevant traits within the biological systems. This information is then stored within the question and search methods database 108a.

One potential use for the user profile 107 is to allow a user to export the user profile to additional (third party) applications or software. This ensures that the genetic and environmental factors that were used to generate the user profile 107 are not transferred to the external software, but the overall profile can still be used to generate other useful data or recommendations.

Certain traits may not be required for computing recommendations, as the traits and systems that are modelled are dependent on the user's goal. The system is given knowledge, either known or learnt, of the relevant traits and biological systems that are relevant to the goal of the user and the system subsequently selects said relevant traits and biological systems to be processed and analysed in the trait and biological system model. The weighting provided to the traits may be altered or reordered based on the knowledge obtained by the system. This, in turn, effects the trait profile generated for the user and the wellness regime and/or recommendations provided by the system.

Trait scores 105a and biological system characterization (also referred to as trait profiles) 106a are analysed within the trait and biological system model 108b using customised algorithms or AI systems which leverage understandings of the relationships, interactions and compound effects between the traits and biological systems specific to the goal of the user. These compound relationship weightings based on the specific user profile 107 and goal 108 are then used to develop personalised wellness (health, diet and fitness) regimes/programs 110 that may be utilised to improve the overall health of the user by implementing changes in fitness, diet and environment.

The output of the trait and biological system model is used in conjunction with a 'fetch' process 109 that matches the relevant trait scores within the relevant biological systems to the appropriate diet and fitness plans. These scores are used to query the diet, fitness and wellness database 109a 109b 109c of recommendations which may prepare a template, by applying values and settings to all of its dynamic elements to arrive at a diet, fitness and wellness recommendation plan that is delivered through a user interface 110 based on the compound interactions and scores from the trait and biological system model 108b.

Along with the user's personalised fitness, diet and wellness plans, the user may also be provided with a personal profile and online interactive graphical interface with one or more mobile applications/devices to track and monitor their progress throughout their plans. As the user progresses through their health regime and experiences changes in their overall fitness and health, their diet, fitness and wellness plans may then be updated by re-running the traits and biological system model in a sandbox environment 112 to produce updated scores and subsequently new and updated diet, fitness and wellness plans based on the progress data reported by the user 111. In a similar manner to that described above, the sandbox 112 may have a sandbox databank 103' and a refined sandbox databank 104', sandbox calculation 103', sandbox trait scoring algorithm 105', sandbox trait scores 105a', sandbox analysis 106' and sandbox trait profiles 106a'. These sandbox elements 103' - 106a' operate in an analogous manner to corresponding elements 103-106a, but allow the system to analyse the effect of changes in the progress data reported by the user 111 and to feed these back into the live corresponding elements 103-106a as necessary.

The mobile health devices and other applications may continue to be utilised to report new user data once the user has begun to implement the diet and fitness programs, and this new data can then be used by the system to compare with the original user data to determine if the user is implementing the diet and fitness programs and achieving their goals. The new and original data may be displayed on the dashboard in graphical or other visual forms to help the user easily view their progress toward their goals related to the diet, fitness and wellness programs. The system does not generate a 'one-off' report; by continuous monitoring and obtaining continuous feedback from the user 111, the diet and fitness programs may be continually modified.

### Data Collection

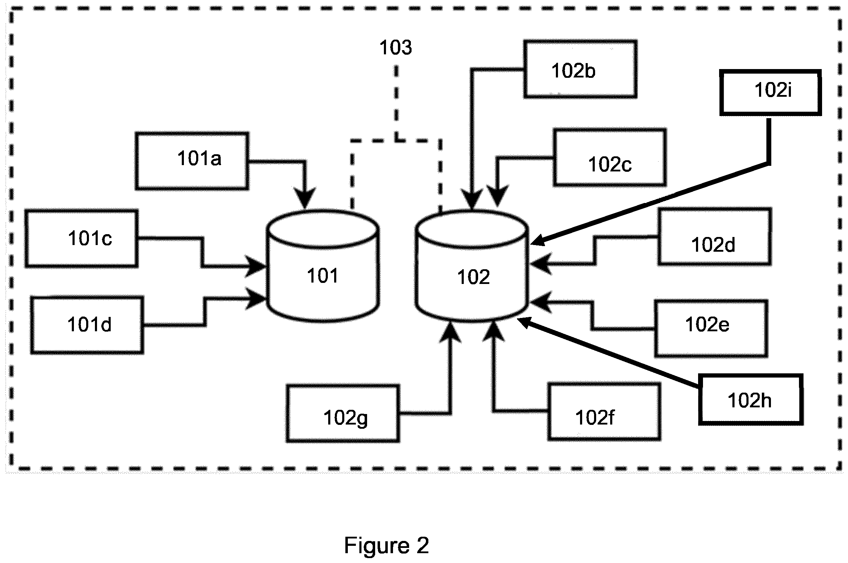

In one embodiment, as seen in Figure 2, user-specific genetic 101 and environmental data 102 are collected from genetic 101a and environmental 102a collection sources. These sources may include, but are not limited to:

### Genetic Data 101

- DNA test 101b
- DNA collection tests 101c
- Medical genetic lab data 101d

### Environmental Data 102

- Health and fitness applications 102b
- Questionnaires 102c
- Mobile Devices 102d
- Environmental lab and medical data 102e
- Health and fitness monitors 102f
- Computing devices 102g
- Health records 102h
- Microbiome data 102i

In another embodiment, the data sources which collect and transmit data on the user may include, but are not limited to:

- BMI monitor
- Glucose monitor
- Blood pressure monitor
- DEXA scans
- FitnessGenes user questionnaires
- Third party health and fitness questionnaires
- Psychological or behavioural questionnaires or tests
- General health check reports from medical professionals
- Pedometers
- Nutritional tracking applications - e.g. MyFitnessPal™
- Apple Health™ / Samsung S-Health™
- FitBit™

These devices/apps are only exemplary, and multiple other genetic, fitness and environmental monitoring devices may be used to generate user data and transmit data directly to the databank 103. Many wireless health devices have open application programming interfaces (APIs) which allow them to be easily integrated with a system running on the front-end cloud server that will compile, analyse and display the data.

In addition to the devices, additional user data 100 can be collected in the form of genetic data 101 from a genetic report from either first or third party 101b 101c or lab results from lab tests 101d that the user has undergone.

Further, mobile devices and health monitors 102d 102f can be configured to continually collect and report real-time environmental data to the databank so the user profile can be continually updated to provide temporally relevant information about the user's diet and fitness progress.

**Trait Scoring**

In one embodiment, a trait is a physiological, behavioural or biological readout; for example, resting blood triglycerides level, resting blood glucose, average blood testosterone level, oxidative muscle dominance, body fat, etc. A trait does not predict a goal or a recommendation, it is a predictive readout of how an aspect of a user's biological or physiological system is at a specific point in time.

Even without any genetic or environmental data added to the system, the system can be initialised so that the trait gives a default score. This can be, for example, the average for the general population. Each trait score may be calculated/modified by the genetic and environmental data that has been collected for an individual user.

How the environmental and genetic factors modify a trait can be highly complex and can require meta-directional analysis which allows for compound gene-environment interactions.

Further, each algorithm that calculates each individual trait generally differs with concentration, transformation, and model-based integrations being used to standardise data. Once the environmental and genetic data has been inputted, modelled and characterised within the algorithms, the trait score can then be modified, giving a personalised readout for that specific trait.

As multiple data types may be collected from different sources, there may be redundancy in the data, with some data modifying or overriding other data points. For example, questionnaires that ask individuals to define their body composition, and enter values for their waist circumference, height and weight. This information can be used to predict body fat percentage but may be overwritten / overridden when data defining actual body fat percentages (from more accurate scanning technologies) is present.

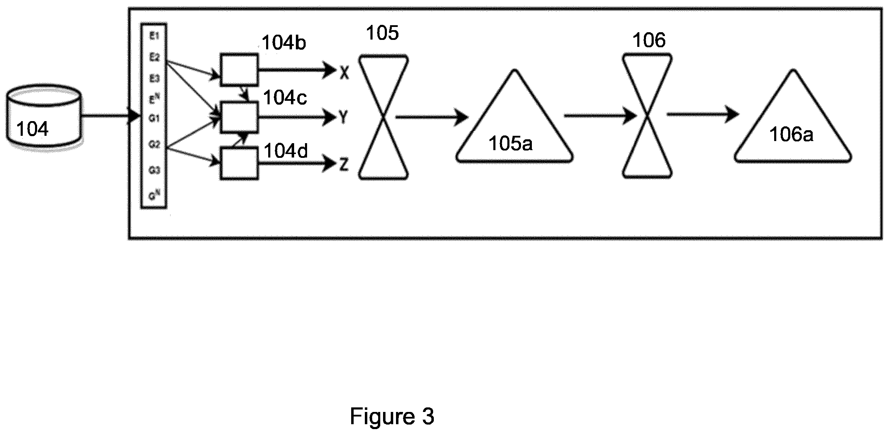

Further, as shown in Figure 3, selected environmental and genetic data points can be integrated and used to modify the trait score 105a to personalise the readout for an individual. The calculations take into account genetic interactions (GxG) 104d, environmental interactions (ExE) 104b and gene-environment interactions (GxE) 104c. Algorithms may involve concentration, transformation, and model-based integrations, as they may deal with multiple types of data.

In this embodiment, trait scores give readouts for a physiological understanding of biological systems 106a. One example of the analysis of the biological systems 106a can include, in an embodiment, analysing the trait scores 105a and aligning the trait scores 105a into one or more trait bands. The trait bands provide a series of bandings or categories that the trait scores align against. Each trait band is indicative of a particular trait outcome. For example, trait scores may be deemed or aligned to be very high, high, medium, low or very low for a particular trait. Such bands provide an indication of the relative expression of the trait. Directionality, namely whether a trait contributes or prevents/acts-against an outcome can also be taken into account.

For example, for the trait testosterone five bands may be provided, ranging from very low to very high, where each band is indicative of predicted current and/or future testosterone levels at current activity levels and/or at expected activity levels if the user implements the wellness regime optimised to their goals suggested by the method.

The use of bandings rather than precise values helps to prevent individual results from skewing recommendations. Continuing with the testosterone example, a very high or very low testosterone trait score may impact upon the method rejecting an otherwise suitable wellness regime or may also have the effect of suggesting a routine based on the very high relative trait score of a single trait, rather than the combination of traits, which is the intent of the system.

| RULE | GENDER MALE/FEMALE/BOTH | DATA ELEMENT | RESULT LOW | RESULT HIGH | Logic 2 (IF APPLICABLE) | AND/NOT/ANDNOT/OR/ORNOT | DATA ELEMENT 2 | RESULT LOW 2 | RESULT HIGH 2 | RULE VALUE ADD/SUBTRACT/DIVIDE/MULTIPY | RULE ACTION |
|---|---|---|---|---|---|---|---|---|---|---|---|
| 1 | FEMALE | SEX | F | F | | | | | | | EXIT |
| 2 | MALE | TEST-TESTOSTERONE | 0 | 200 | | | | | | -5.5 | EXIT |
| 3 | MALE | TEST-TESTOSTERONE | 200 | 400 | | | | | | -2.5 | EXIT |
| 4 | MALE | TEST-TESTOSTERONE | 400 | 600 | | | | | | 0.0 | EXIT |
| 5 | MALE | TEST-TESTOSTERONE | 600 | 800 | | | | | | 3.0 | EXIT |
| 6 | MALE | TEST-TESTOSTERONE | 800 | 9999 | | | | | | 4.0 | EXIT |
| 7 | MALE | TEST_2_rs6258 | TT | TT | | | | | | 0.8 | CONTINUE |
| 8 | MALE | TEST_2_rs6258 | TC | TC | | | | | | 0.0 | CONTINUE |
| 9 | MALE | TEST_2_rs6258 | CC | CC | | | | | | 1.0 | CONTINUE |
| 10 | MALE | TEST_3_rs722208 | GG | GG | | | | | | 0.0 | CONTINUE |
| 11 | MALE | TEST_3_rs722208 | GA | GA | | | | | | 0.8 | CONTINUE |
| 12 | MALE | TEST_3_rs722208 | AA | AA | | | | | | -1.0 | CONTINUE |
| 13 | MALE | TEST_rs1799941 | AG | AG | | | | | | 0.5 | CONTINUE |
| 14 | MALE | TEST_rs1799941 | AA | AA | | | | | | 1.0 | CONTINUE |
| 15 | MALE | TEST_rs1799941 | GG | GG | | | | | | 0.0 | CONTINUE |
| 16 | MALE | ACTN3_rs1815739 | TT | TT | | | | | | 0.0 | CONTINUE |
| 17 | MALE | ACTN3_rs1815739 | TC | TC | | | | | | 0.5 | CONTINUE |
| 18 | MALE | ACTN3_rs1815739 | TT | TT | | | | | | 1.0 | CONTINUE |
| 19 | MALE | BODY Type | UNF | UNF | | NOT | BODYFAT | 0 | 99 | 1.0 | CONTINUE |
| 21 | MALE | BODY Type | UNW | UNW | | NOT | BODYFAT | 0 | 99 | -1.0 | CONTINUE |
| 24 | MALE | BODY Type | OBS | OBS | | NOT | BODYFAT | 0 | 99 | -5.0 | CONTINUE |
| 25 | MALE | BODY Type | OVW | OVW | | NOT | BODYFAT | 0 | 99 | -2.0 | CONTINUE |
| 27 | MALE | BODY Type | AVG | AVG | | NOT | BODYFAT | 0 | 99 | 0.0 | CONTINUE |
| 28 | MALE | BODYFAT % | 26 | 100 | | | | | | -5.0 | CONTINUE |
| 29 | MALE | BODYFAT % | 19 | 26 | | | | | | -2.0 | CONTINUE |
| 30 | MALE | BODYFAT % | 15 | 19 | | | | | | 0.0 | CONTINUE |
| 31 | MALE | BODYFAT % | 7 | 15 | | | | | | 1.0 | CONTINUE |
| 32 | MALE | BODYFAT % | 0 | 7 | | | | | | 0.0 | CONTINUE |
| 33 | MALE | AGE | 60 | 200 | | | | | | -2.0 | CONTINUE |
| 34 | MALE | AGE | 45 | 60 | | | | | | -1.5 | CONTINUE |
| 35 | MALE | AGE | 30 | 45 | | | | | | -0.5 | CONTINUE |
| 36 | MALE | AGE | 0 | 30 | | | | | | 0.0 | CONTINUE |
| 37 | MALE | ACTIVITY | VA | VA | | NOT | MET_HOURS | 151 | 670 | 1.0 | CONTINUE |
| 38 | MALE | ACTIVITY | A | A | | NOT | MET_HOURS | 151 | 670 | 0.5 | CONTINUE |
| 39 | MALE | ACTIVITY | M | M | | NOT | MET_HOURS | 151 | 670 | 0.0 | CONTINUE |
| 40 | MALE | ACTIVITY | L | L | | NOT | MET_HOURS | 151 | 670 | -1.0 | CONTINUE |
| 41 | MALE | MET_HOURS | 320 | 670 | | | | | | 1.0 | CONTINUE |
| 42 | MALE | MET_HOURS | 301 | 320 | | | | | | 0.5 | CONTINUE |
| 43 | MALE | MET_HOURS | 234 | 301 | | | | | | 0 | CONTINUE |
| 44 | MALE | MET_HOURS | 205 | 234 | | | | | | 0.2 | CONTINUE |
| 45 | MALE | MET_HOURS | 178 | 205 | | | | | | -0.8 | CONTINUE |
| 46 | MALE | MET_HOURS | 151 | 178 | | | | | | -1.0 | CONTINUE |

Table 2. Logic for the trait testosterone

Table 2 details the logic built for the testosterone trait. Each row details a rule that 1) has an assigned gender, 2) has a series of condition rules that comprise of 'Data Elements' and results/values ('Result High' and 'Result Low'), and 3) a rule score of that condition set ('Rule Value'). Conditions rules are linked together via logic, and any number of condition rules can be applied to a condition set assigned to a rule. The rule value of each rule is taken into the trait calculation if the conditions of the rule are satisfied. The values taken forward are then used in the trait calculation. Rules values can be added together, or can be entered into more complex calculations. In this example, the values are added together. The 'rule action' details where the information from a rule is enough to satisfy the trait. If satisfied an 'Exit' action is applied.

For example, in the testosterone trait score calculation example, if the system encounters the factor or data element of the user being female then the trait score is null and the trait calculation ends. Similarly, if the (male) user provides an external testosterone blood test level as a data element or factor, then the level of the testosterone blood level result is compared to the conditions according to the banded levels shown. For example, a blood testosterone level of between 0 and 200 results in a trait score of -5.5. Here, because the data input is a known accurate reading, the trait calculation ends.

For rules 7 to 9 of Table 2, a genetic data element is analysed to determine which gene phenotype is expressed. For types TT or TC, the rule value returns a score of 0.0 and the calculation continues. For type CC, a score of 1.0 is (in this example) added to the current or a base trait score.

Similarly, data elements may be combined. This is shown in rules 19, 21, 24, 25 and 27 where the data element relating to BODY Type is linked with the data element BODYFAT. In the example shown, the body type (which is self-reported by the user) is used if the data element BODYFAT is unavailable. It can be appreciated that the bodyfat % data element is generally a more accurate data element for testosterone calculation than body type. Other trait score contributing factors shown in table 2 relate to age, activity and the number of active hours per week (Met_hours). From a combination of the condition rules a trait score can be determined for each trait. It can be appreciated that although the above relates to the testosterone trait, similar conditions and condition sets can be generated and used for other traits.

The trait information may be informative for scientists and healthcare professionals; but in this embodiment they do not by themselves offer recommendations. The trait scores are used in combination to infer higher level understanding of an individual's specific biological systems 106a, and ultimately may allow for actionable recommendations to be made.

It can be envisaged that the trait information could be provided to external parties in a controlled 'black-box' arrangement where the system or method compiles information provided to and supplied from the system or method from third parties, whilst shielding such third-parties from the trait-trait interaction and scoring definitions.

Implementation options include:

- The genetic testing platform may not limit the number of traits in the system.
- Traits may be modified, or even be split into multiple traits.
- Traits may not be exclusive, and may interact when being used to derive recommendations, or when modelling more complex biological functions or phenotypes.
- Genetic data can be from more than one platform including third party data sets.
- Environmental data may encompass all other non-genetic data.

## Trait and Biological System Model

Once the trait scores for an individual have been calculated and utilised to understand the physiological and behavioural makeup of the biological systems of the user, it may be possible to begin the recommendation process. Trait scores can be fixed at any given point in time, the fitness, diet and wellness plans given to the user are then based on the hierarchical compound weighting of traits associated with particular goal-linked questions and their effects on the biological systems to produce a readout figure. That is, the question (which defines a goal) has knowledge of which biological systems and traits are relevant to achieving the goal and the weighting and interactions between traits within the biological systems. The fetch process then matches the relevant traits within the relevant biological systems to the appropriate diet, fitness and wellness plans.

There may be various ways of defining the hierarchy of the traits and the interaction between them. Some of the methods for calculating the hierarchy include, but are not limited to, manually defining the hierarchy or computing said hierarchy by AI based upon collected data that has been analysed.

Different traits may affect different biological systems within the body and may affect more than one system at any one time. The system generally has knowledge about the compound effects and interactions any one or more trait can have on one or more body system for any one or more scenarios which may depend upon the goal of the user. The knowledge may be hardcoded, or may change and be refined over time, representing the current estimate based on the data currently analysed (include literature/AI based analysis). Further, the biological system can be characterised by a prediction calculation based on the traits of the user including the compounding effects of trait-trait interactions at the biological system level.

In such an embodiment, predefined and tailored recommendations and search methods based on what a user wants to achieve are used. Some trait scores and biological systems may not be relevant and thus may be excluded as the specific traits relevant may be based on the user's goals and thus, a selection within the system to pinpoint which traits and biological systems are relevant to the question may take place.

Accordingly, in this embodiment, which is complimentary with earlier embodiments, the system or method can also determine one or more recommendations, said recommendations providing an actionable outcome for the user. The recommendations can be determined based on one or more of: trait scores; and/or trait profiles; and/or data elements. Data elements, which will be described in greater detail below typically comprise one or more genetic factors, and/or one or more environmental factors and/or one or more wellness goals.

Data elements are intended to provide extra resolution to the recommendations, but are not classed as traits in themselves but instead are context to a trait score. For example, based on a trait defining omega 6 intake (or omega 3 to omega 6 fat ratio) plus a data element describing diet the system or method can determine whether a recommendation is made for a user to alter their omega 6 intake. Due to the contribution of the data element 'diet', then the system can determine a sensible recommendation on how to do this based on their diet i.e. if they are vegetarian, a recommendation isn't made to eat more fish.

As an additional example, a recommendation may be provided to the user regarding the total amount of protein that should make up the users diet. This may be provided as a % of food intake, kcal intake, or a goal intake of protein in grams. The recommendation may be given to the user in bands, ranging from very low to very high. The recommendation level or band selected in this instance depends on the trait scores for the traits describing satiety, insulin sensitivity, obesity risk and lean body mass. Non-trait data elements that are relevant to determining the banding or level of recommendation for this example include age of the user, intermediate/active/ultimate goals of the user and goals of the selected wellness regime.

Although only two example recommendations have been described it can be appreciated that numerous recommendations could be generated.

In a further complementary embodiment, like with the trait scores and banding, the recommendations may be weighted depending upon the trait scores or trait bands or data elements which influence the recommendation. For example, a recommendation on whether to supplement the user's diet with vitamin B12 that is dependent on the trait describing folate metabolism and the environmental factor diet is likely to be heavily weighted to diet. It can be appreciated that other weightings may be appropriate for other recommendation. Bandings may also be used to highlight the relative strength or positively recommended effect of following the recommendations.

In another embodiment, when the overall system is queried as to what fitness regime or diet would meet a particular desired goal, an algorithm may be used to match training/diet advice with the user's specifically defined biological system and trait profile, including the compounding effects of trait-trait interactions at the biological system level which will produce a trait profile in order to create recommendations for a user. To do this, questions are set up within the system 108. For each question, the system has the knowledge and the tools to identify the hierarchy between the traits and to determine which traits are relevant to the question.

This means the system may take the traits required for that specific question, and identify the interaction and hierarchy between them. For example, when recommending macronutrient splits, an individual's 'resting blood triglycerides level' and 'resting blood glucose' traits may impact on the recommendations for dietary fat percentage and carbohydrate percentage respectively. In addition, as these percentages of macronutrients contribute to an individual's overall calorie intake, these changes may impact on the relative amount of protein in the diet. Furthermore, another trait, 'appetite suppression', may also alter the protein percentage in the diet. This means that within the system, the interactions and hierarchies may be set out correctly for each question. This may then create a final set of scores from the traits (including the compounding effects of trait-trait interactions at the biological system level that will allow for the generation of the personalised recommendations).

**A detailed embodiment**

In this particular embodiment, the system matches predefined goal-focussed questions with pre-defined wellness, training and/or nutrition-related answers which have been hardcoded based on industry knowledge.

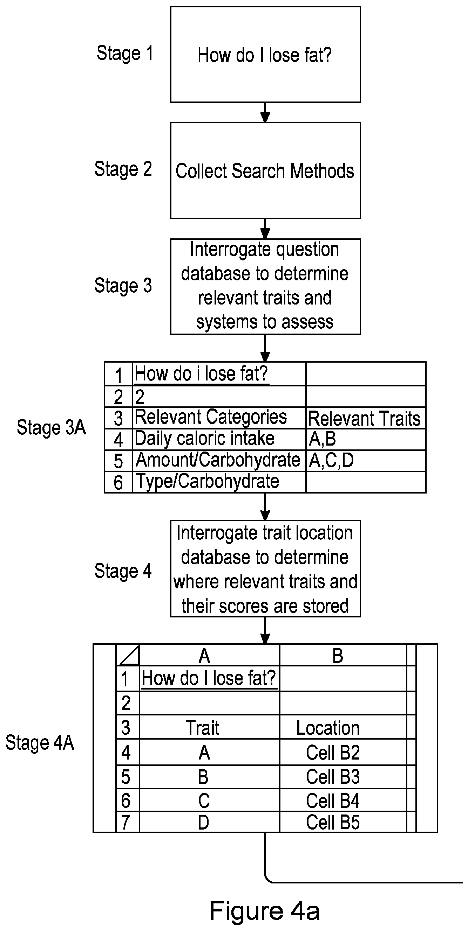

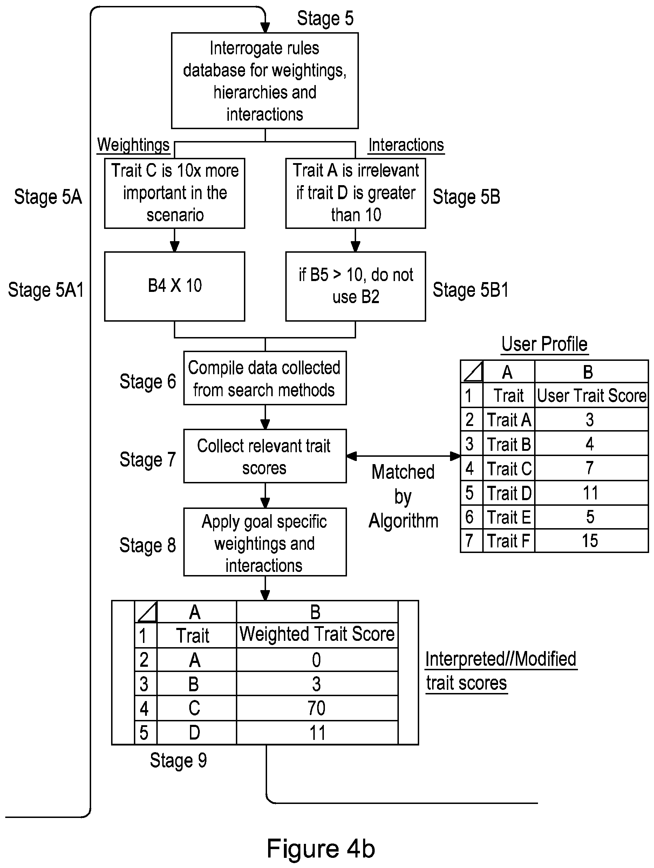

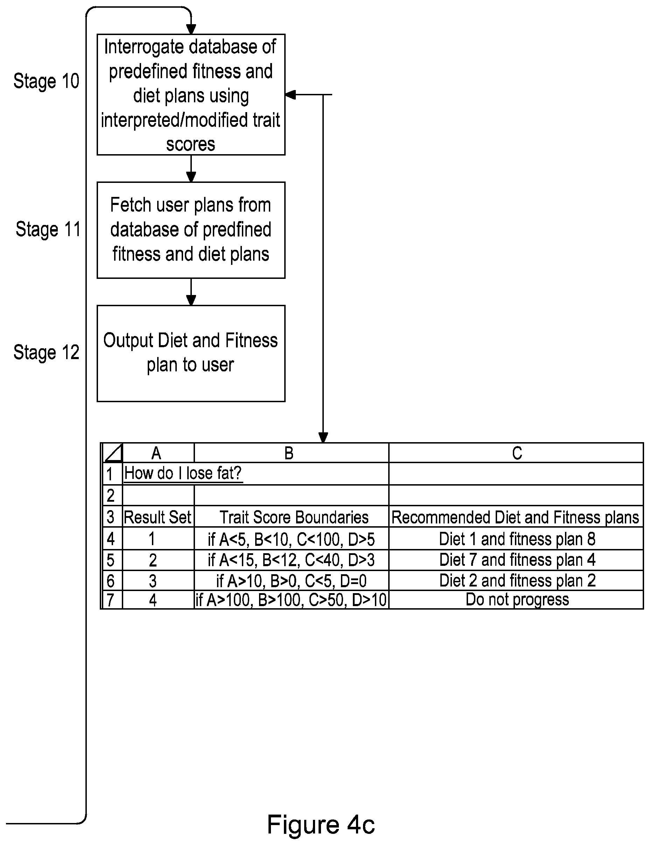

As shown in Figure 4, the relevant stages for producing goal related wellness, fitness and diet programs for a specific user, based on their genetic makeup and environmental data may be detailed below.

In Stage 1, the user may decide upon the relevant goal they wish to pursue and select this fitness and diet related goal within the system.

For example, a user may select *"How do I lose fat?"*

For the purpose of this embodiment, we may define the following:

- **Search methods** - data pertaining to:

   o The relevant biological systems and traits to be assessed based on the goal
   o The relevant weightings and interactions of those traits
   o The location within the system of those user specific traits and thus, trait scores

Upon selecting that specific goal-related question, the system must be interrogated to extract the knowledge and search methods required to produce a diet and fitness recommendation for the goal-related question of the user.

Note: For the goal-related question in Stage 1, and other goal-related questions in other embodiments, the search methods may be predefined within the system prior to the question being asked. This means, the system may have predefined knowledge of all the search methods, for every possible question a user may be expected to ask of the system (or for questions provided to the user by the system) and thus, when a goal-related question is selected and the interrogation begins, the relevant database may output the specific search criteria. Tagging search methods to certain goal-related questions may be necessary as different goals may involve different traits, different interactions between the traits and be relevant to different biological systems of the user.

Each goal-related question is tagged with the necessary search information to find, order and prioritise relevant traits within the biological systems of the user. The system is instructed to begin gathering this information in Stage 2 of Figure 4 where it starts collecting the goal-related, predefined search methods from their relevant databases. For the purpose of this embodiment, the search methods are located within the following databases and may be collected using a fetch algorithm:

- Question database - containing relevant biological systems and traits
- Trait location database - containing traits tagged with their specific locations within the user profile
- Rules database - containing goal specific weighting and interactions to be applied

The question database, as shown in Stage 3A contains relevant recommendation categories that encompass the relevant biological systems and data stating which traits are relevant to each category for a specific goal-related question. For every goal-related question within the system, the question database within Stage 3A will contain the following:

- List of relevant recommendation categories
- List of traits that are relevant to that specific recommendation category

Note: The question database in Stage 3A may be produced based on industry knowledge, academia, 1st and 3rd party research and other sources. It provides a non-user-specific overview of which traits are relevant to which category for a specific goal-related question.

In this embodiment, the database has informed the system that the relevant categories that need to be assessed are "daily caloric intake", and "carbohydrates" among others. For the purpose of this example, the categories to be assessed can be the "daily caloric intake" and "amount of carbohydrate" categories. Within the "daily caloric intake" category, the database has recommended that the traits that need to be assessed and are relevant to "losing fat" are traits A and B. Within the "amount of carbohydrate" category, the database has recommended that the traits that need to be assessed and are relevant to "amount of carbohydrate (to be consumed on daily basis in diet)" are traits A, C and D.

Note: the "daily caloric intake" and "amount of carbohydrate" categories and subsequent traits may not be the only relevant category to be interrogated for this specific goal, but for the purpose of this example embodiment, it is the only system computed and analysed here. A more thorough example of categories available is in table 1 below:

| RECOMMENDATION CATEGORIES | FAT LOSS RECOMMEND ON: | RELEVANT TRAITS |
|---|---|---|
| Daily Calorie Intake | Total calories | A, B |
| Carbohydrates | Ratio and amounts, types, periodisation | C, A, D |
| Fibre | Sources and importance for weight loss | Etc. |
| Sat Fats | Ratio and amounts | |
| Mono Fats | Ratio and amounts | |
| Poly Fats | Ratio and amounts | |
| Total Fat | Ratio | |
| Protein | Ratio and amounts, timing of intake | |
| Micronutrients | RDAs, food sources, vitamin D/lactose/antioxidants | |
| Meal Frequency | Yes | |
| Caffeine | Impact on fat burning? | |
| Lactose | Advice on intolerance | |
| Hydration | Importance for fat loss and recovery | |
| Sodium/Salt | | |
| Magnesium | | |
| | | |
| EXERCISE | | |
| Type | Resistance and cardiovascular (HIIT, steady state,) home based | |
| Frequency | Days per week of resistance and cardio, body splits | |
| Intensity | %VO2 max/HRmax | |
| Sets/Reps | Sets and reps for both HIIT, resistance training | |
| Rest | between sets and workouts, recovery strategies | |
| Duration | Specified cardio duration in mins/interval durations, total weeks | |
| Timing | Weekly and time of day | |
| | | |
| SLEEP | Recommended amounts, importance for fat loss, strategies to improve sleep | |
| | | |
| STRESS | | |
| | | |
| SUPPLEMENTS | Caffeine, Green tea catechins | |

Table 1: Categories related to a goal/question of fat loss.

In the example shown in Table 1, the category 'daily calorie intake' is associated with traits A and B. In this example, trait A corresponds to a user's resting metabolic rate, whilst trait B is the user's activity level(s). As noted above, the information for these traits may be obtained from external sources (such as a fitness/movement monitor for activity levels), external applications, questionnaires or the like.

For category 2: carbohydrates, there are a number of elements or variables on which recommendations can be made, such as the 'amount' or 'type' of carbohydrate. In this instance, with regard to the 'amount' of carbohydrate to be recommended, the traits 'insulin sensitivity' (C), 'resting metabolic rate' (A) and 'blood triglycerides levels' (D) are considered relevant. The traits relating to all relevant elements for each category make up the 'trait profile' for that category.

Once the system has knowledge of which categories and traits are relevant, it must then interrogate a trait location database - Stage 4 - in order to obtain the necessary location information for a user's traits and in turn, trait scores. Location information, in this case, may be described as a specific point within the user profile that contains the relevant trait scores for the goal-related question.

The system needs to know where to fetch the trait scores from; this knowledge is passed to the system when it interrogates a trait location database in Stage 4A, again using a fetch algorithm. The system knows from Stage 3A which traits it must select, thus, when it has located the relevant traits it collects and stores the traits (which are tagged with their locations in the user profile) to be used in the subsequent stages.

For the purpose of this embodiment, the trait location database shown in Stage 4A indicates that trait scores for traits A, B, C and D, the ones relevant to "losing fat", are shown to be stored within the following locations within the user profile (shown in stage 7A):

- Trait A - Cell B2
- Trait B - Cell B3
- Trait C - Cell B4
- Trait D - Cell B5

Note: The system has detailed, quantified physiological knowledge and understanding of the user in the form of trait scores. These scores have been calculated based on the environmental and genetic data collected and are stored in a known location within the user profile. The trait location database in Stage 4A stores this location information for a user's trait scores.

Next, within Stage 5, the system must interrogate a rules database to obtain knowledge of the relevant goal-related trait weightings and interactions on the biological system level.

Note: That means, the specific weightings and interactions that need to be applied to Traits A, B, C or D for the "daily caloric intake" and "amount of carbohydrate" categories based on the question of "losing fat".

In this embodiment, these weightings and interactions may be predefined.

For example, for daily caloric intake: Each trait has a score e.g. resting metabolic rate (GxE) = n1 = high; activity levels (ExE) = n2 = high and a set of logic/ rules / algorithm exist that determine how to interpret each combination of scores together for each category on a biological systems level e.g. daily calorie intake = 2500. This gives an output that is assigned to a particular outcome 'band' with associated recommendation e.g. eat 2500 calories a day.

Similarly, for amount of carbohydrate (as above): Trait C = (n3) = poor, Trait A = (n1) = high, Trait D = (n4) = high, e.g. the recommendation may be to reduce amount of carbohydrate in the user's diet relative to an average level by xx grams, where xx is the recommendation determined for the user.

Categories cannot be considered in isolation however, as there may be contradictions or contraindications. and thus they must be considered in combination - the multi-level biological systems approach, exemplified in Stage 8.

For example, how does daily calorie intake impact on: carbohydrate vs protein vs fats, and how does carbohydrate amount impact on: daily caloric intake vs protein vs fats, etc.

For example, if calories are >2500, which may reflect an increased endurance activity level, the system can determine and advise the user to hold protein at a maximum ratio of 30% in terms of macronutrient ratio, and to increase carbohydrate ratio respectively.

In another example, if the output of 'amount of carbohydrate' in diet is 'reduce' and the output of 'amount of fat' in diet is 'reduce' the consequence on total macronutrient ratio is a significantly increased 'amount of protein' in diet. However this may not be an optimum health recommendation, as high protein can be linked to certain health issues. As a result, categories need to be prioritised appropriately. In this instance, the trait 'insulin sensitivity' may be considered to be more important than other relevant traits and as a result, the output for 'amount of carbohydrate' is prioritised above 'amount of fat'.

*Stage* 5A *and* 5A 1

For a trait relevant to a certain goal related question, a weighting may be applied to the trait score of a user. For example, there may be scenarios where, due to the goal, a specific trait score is required to be weighted more heavily within one or more categories.

In this embodiment, as seen in Stage 5A, Trait C is seen to be 10x more important in the context of the "amount of carbohydrate" category for this goal-related question. Once the system has obtained that Trait C must be magnified by a factor of 10, it must also obtain the relevant code and/or equation for applying this weighting. This code may also be stored within the rules database and collected by the system in Stage 5A1.

*Stage 5B and 5B1*

For traits relevant to a certain goal related question, an interaction value may be applied to the trait scores of a user due to hierarchies within traits. For example, there may be scenarios where, due to the goal selected, a specific trait score becomes irrelevant due to a score of a different, or "overpowering" trait that takes priority.

In this embodiment, as seen in Stage 5B, Trait A is seen to be irrelevant if Trait D has a larger value than 10 for this goal-related question. Once the system has obtained that Trait D must be ignored if Trait C is greater than 10, it must also obtain the relevant code and/or equation for applying this interaction. This code may also be stored within the rules database and collected by the system in Stage 5B1.

Upon completion of Stage 5, the system may compile all the search method criteria which can be used to model the relevant data based on the goal-related question, this is performed in Stage 6.

Once this has been compiled and computed, it may have the following knowledge and be able to progress to the modelling and recommendation stage:

- Knowledge of which goal-related question the user has chosen - Stage 1
- Knowledge of which recommendation categories and subsequent traits of the user are relevant to that goal-related question - Stage 3A - which it has gained from interrogating the question database in Stage 3.
- Knowledge of where to obtain the relevant trait scores for the user - Stage 4 and 4A
- Knowledge of the relevant goal-related trait weightings and interactions on the biological system level to be applied to the user's trait scores - Stage 5

Note: The user profile contains the trait scores along with a detailed physiological understanding of the user and the system now has a complete search methodology for the goal related question of "losing fat" as defined in the stages above.

In Stage 7, the system uses the search methods compiled in Stage 6 and fetches the relevant trait scores using a predefined algorithm from within cells B2, B3, B4 & B5 in the user profile - Stage 7A.

For this embodiment, these scores are:

Trait A = 3
Trait B = 4
Trait C = 7
Trait D = 11

The system stores these scores to be used in Stage 8 where the relevant weightings and interactions are to be applied.

Note: The trait scores detailed above for this user may be fixed at this given point in time as they are based on the environmental and genetic data collected, analysed and computed to produce the user profile. These trait scores will be used to apply the relevant goal-related weightings and interactions computed in Stage 6.

Note: The overall output may be based on the weightings of these trait scores along with the trait-trait interactions on a biological system level. These outputs will change relevant to the question and relevant to the initial trait scores of the user.

In this embodiment, using the user trait scores collected in Stages 7 and 7A, the specific weightings and interactions to trait scores A, B, C and D may be applied using an algorithm. This process occurs in Stage 8 of Figure 9. These weightings may be dynamic and change dramatically depending on the goal of the user but for this case, the weighting and interactions for "losing fat" have been computed within Stage 5 of the system and the calculations are follows:

Weighting:

$$\text{Trait C x 10} = 7 \text{ x } 10 = \underline{\textbf{70}}$$

Interactions:

If Cell B5 > 10, Cell B2 = 0
Trait D in Cell B5 = 11,
Therefore: **Trait A in Cell B2 = 0**

Applying these weighting and interactions to the user's initial trait scores changes the value of the scores relevant to the search methodology computed for goal-related question of the user in Stages 3, 4 and 5. An algorithm may be used to apply the trait weightings and the trait-trait interactions as described above and then outputs a data set of interpreted/modified trait scores which the system can use to begin the recommendation process for diet, wellness and fitness plans. This output may be considered to be a trait profile that determines or defines the biological system level for that user. Such profile or biological system level is therefore a representation of the interaction between traits within categories e.g. the amount of carbohydrates recommendation linked to the metabolome and endocrine systems represented by the traits listed.

The interpreted and modified trait scores are stored within a database and this can be seen at Stage 9 of figure 4.

Note: In this embodiment, only interactions and weightings relevant to the goal-related question may be modelled. It may be noted that, multiple weightings and interactions may need to be applied for a specific goal-related question but for the purpose of this embodiment, a single weighting and a single interaction has been used. The algorithm may also search in between systems and analyse at the effects that systems have on each other at both system and trait level and these effects may further be used to modify a user's trait scores.

The system will now have user specific, goal-related interpreted/modified trait scores that encompass the relevant trait weightings and trait-trait interactions computed by the algorithm.

These scores are used to make a diet, wellness and fitness related decision for the user.

In this embodiment, the system may query a database (Stage 10) of pre-defined goal specific recommendation category ranges (Stage 10A) that are utilised to direct the system towards a certain diet, wellness and exercise routine. The system may utilise the modified and interpreted scores from Stage 9 to compare, using true/false match algorithm, the users' scores to the predefined score ranges within the database to output the most relevant and beneficial diet and fitness plans.

Note: For example, the database may contain the following:

If Trait A less than 5, Diet plan 1
If Trait A is 5 or more, Diet plan 2.

It can be appreciated that more complex database elements may be used, for example, recommendations may be implemented as described above and below.

The system may compare the users modified trait scores to the ranges defined in the database and fetch the diet, wellness and fitness plan that is tagged to that range. If the users score for Trait A is 4, the system will fetch diet plan 1.

In this embodiment, these ranges will be predefined and the final plans may be manually produced based on industry knowledge, academia, research and that are commonly known recommendations for specific fitness, wellness and diet related goals.

The output from Stage 10A will then be used in Stage 11 where the system may now fetch the numbered diet, wellness and fitness plans from the database of predefined diet, wellness and fitness plans.

These plans will contain the specific fitness regime containing a macronutrient breakdown based on person's goals, genetics, environmental data, hierarchical weightings, trait-trait interactions and system-system interactions which may be sent to the user via a graphical online interface in Stage 12.

**Feedback ability**

The progress of the user can be inputted back into the environmental data to allow the system to update the tailored recommendations. The diet, wellness and fitness recommendations may change any time a user updates or provides feedback to the system.

In addition to customer feedback, new genetic associations and research paradigms can be incorporated to constantly update the system. This may be done using bioinformatic analysis or artificial intelligence.

**Overview**

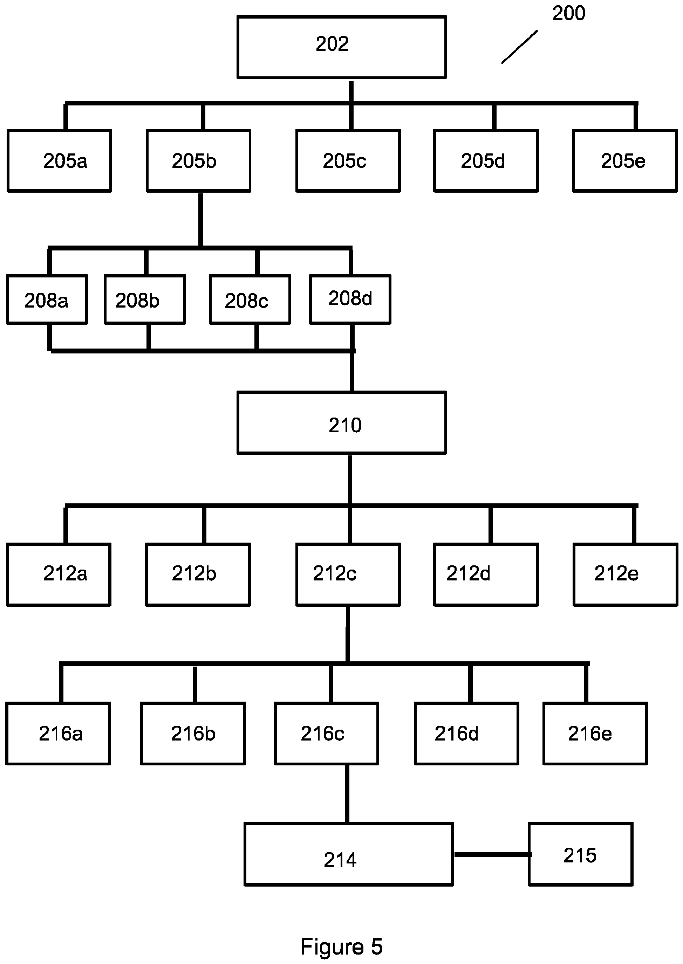

An additional complementary or alternative way of considering the system or method is described in relation to Figure 5. Here, a method 200 of determining a wellness regime for a user is described. The method comprises the steps of determining user information or data elements 202. The information can include goals, questionnaires, genetic data, physiological data and environmental data of the type described in detail above in relation to the other figures. It can be appreciated that the system may obtain only a partial set of data at point 202 and may query and utilize goals or additional data at later points of the method, such as once the trait profile of a user is known.

Once the data elements 202 are received or obtained, the method or system determines a number of traits 205a-e, each trait being a physiological, behavioural or biological states of the user. Again, examples of traits are described above.

Taking trait 205b as an example, the trait 205b has a number of genetic and/or environmental factors 208a-d that are relevant to the trait 205b. Some traits may have only genetic factors, some traits may have only environmental factors, although the majority of traits have a combination of genetic and environmental factors.

It can also be appreciated, that although shown as having four factors 208a-d, a trait may have more of less factors. Each factor typically provides a score, value or range that is indicative of the effect that the factor has on the trait.

As a specific example, for testosterone, genetic variations associated with the genes ESR1, ACTN3, SHBG1 and SHBG2 are considered relevant genetic factors, whilst body type, body fat %, age, activity levels, weekly active hours and sex are relevant environmental factors. The factors are obtained from the data elements 202 obtained from the user.

Once the relevant traits 205 and their associated factors 208 are known a trait score is calculated 210. The trait score is an indication of the degree of expression of the trait for the user. Trait scores can be values or can be a series of bandings 212a-e depending upon the trait and/or the detail or complexity of the trait analysis required. The trait profile can be considered to be the user's biological system or 'biological fingerprint' and provides an indication of the user's likely biological, physiological and/or behavioural response to a wellness regime.

The trait scores are determined by weighting the genetic and/or environmental factors, where the weighting defines the likely effect that the genetic and/or environmental factors have on the trait. The weighting is determined by the system and it may be appreciated that different factors may have differing weightings depending upon the relative values of the other factors. For example, if a user's bodyfat is above a certain threshold value, then all genetic factors may be weighted sufficiently lower such that bodyfat is the key determinant for the trait score. Alternatively, if the user's bodyfat is below a threshold value, or within a bounded series of values then genetic factors may be a key determining factor. The nature of the weighting may be linear, but is often parabolic.

Unknown factors may be approximated based on the other factors, or may be assigned an average value based on other user's data. Similarly, in scenarios where specific values of traits are known, such as the result from a blood testosterone level, then the factors can be disregarded and the actual result or reading used.

The trait score is indicative of a particular trait outcome. This is particularly easy to appreciate where bands are used. For example, a user's testosterone trait, which is a prediction of the user's physiological testosterone levels, may be deemed very high, high, moderate, low or very low depending upon the calculated trait score.

A trait score 210 is calculated for each trait 205 that is under consideration by the system 200.

Trait scores can then be used to generate wellness recommendations 216a-e. The recommendations 216 are generally nutritional (for example, daily calorie intake, protein ratio etc.), supplemental (Omega 3/6 supplementation, creatine supplementation etc.), wellness (# of hours sleep, etc.), exercise (exercise type, frequency, etc.), behavioral or other category of recommendations. Each recommendation 216 is based on a predetermined trait or combinations of traits, optionally with data elements 215.

As with the trait results, the recommendations 216 may be banded, or may be values. As an example, a recommended daily calorie intake is generally a value or number, whilst a protein ratio is generally provided to the user as a banding from very low, low, moderate, high, very high etc. with each band representing a wellness action for the user optimised towards meeting the wellness goals 215.

Traits that do not contribute to the goals 215 of the user may be given a null or zero weighting against any particular recommendation 216. This is particularly the case for traits that are binary - the trait is either relevant or not.

Based on the recommendations, 216 the system can then determine a wellness regime 214 that is tailored to achieving the goals of the user 215. The system may determine that the ultimate goals of the user require the user to first reach an alternative milestone or minimum level. For example, a minimum fitness level may be required to achieve some goals. Alternatively, the system may determine that a user's health or wellness would be better improved by improving the user's cardiovascular health rather than attempting to build muscle (as an example).

As noted above, data elements 202 may also be utilized to add additional context and information to the wellness regime and/or to the recommendations. Data elements 202 are typically one or more genetic factors, and/or one or more environmental factors and/or one or more wellness goals.

It can be appreciated that the system and method 200 described are capable of being dynamically updated in semi-real time such that feedback on the user's response to recommendations 216 and the user's compliance with these, which may be self-reported or may be incoming data from a wellness device or test aids development of the wellness regime 214 and additionally the traits 205 if the incident data 202 or goals 215 of the user change.

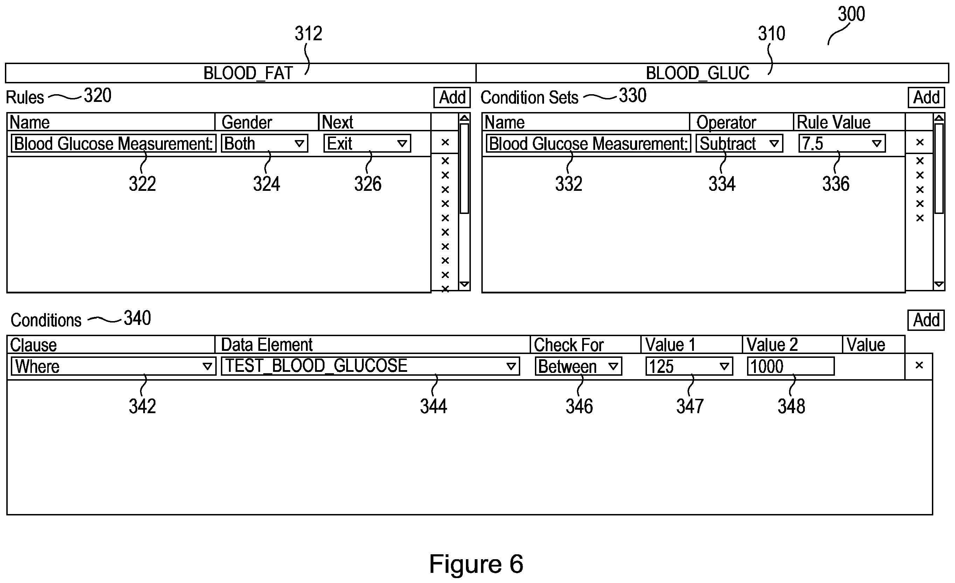

Figure 6 shows an example of a user interface 300. The user interface 300 is intended to be a supplemental option to the embodiments described above. The user interface provides an interface for the implementation and creation of rules, conditions relating to traits and recommendations. This allows additional traits and recommendations to be determined, or existing traits modified accordingly. The user interface 300 has a browser based layout with tabs, buttons and drop down menus.

In the example shown in Figure 6, a tab 310 is provided for the trait 'BLOOD_GLUC'. This rule deals with measurements of resting blood glucose. A further tab 312 for the trait 'BLOOD_FAT' is currently unselected. Within the interface 300, a number of rules can be generated. New rules can be added by selecting the add button 322. In the present example, one rule 324 is shown. The rule relates to blood glucose measurement of the user. Rules may be selected from known rules, or may be created. The rule 324 is shown as applicable to both genders 326, with the additive 328 being that the program is stopped from running if the rule conditions are met.

These rules 320 can be assigned gender, and be either additive ('continue') or stop the program running (exit; this happens when a data element of a rule allows for full predictive capability at that point).

Condition sets 330 can also be defined for each rule 320. In this instance, the condition set is that if the rule 324, 332 is met, then a trait score is returned with an operator 334 of subtract (although addition, divide, multiply could apply) and a value 336 of 7.5. So the condition set has a value of -7.5 associated with it ('subtract 7.5'). The condition set allows for addition, subtraction multiplication or division between rule values.

Each condition set 330 can also have a number of conditions 340. In this instance, a logic operator or clause 342 informs the system that where the data element 344 (data element being data of the selected user obtained as noted above) has a value according to a check for (in this instance between) 346 two value bands 347, 348, then the condition assigns a value according to the condition set 330. So the condition will assign the value of -7.5 to a trait score when a customer has a blood glucose test (Test _Blood_Glucose) between 125- 1000. This is very high. The conditions can be qualitative information, or be values or ranges of values set that allow for addition, subtraction, multiplication or division between rule values.

Values can also be assigned to genetic variants. For example, a value may be assigned if a user has a specific genotype. Traits can also have assigned bands that house ranges of trait scores. A trait band is a range of values that are assigned to one particular trait outcome - for example for 'blood glucose' 'VERY_HIGH'. Values can also be used to assign more precision over time.

From this interface 300, as user or customer data is fed into the system, the system will analyse each rule, with its condition sets and conditions, in turn. At the end a trait score will be calculated. Traits scores and where used trait bands can be used to build recommendations. Recommendations can also have assigned bands that will house ranges of recommendation scores. These can then be assigned to recommendations.

Traits and recommendations can be published to a 'Vault' (stored), to the 'Live Server' where they are used in customer products, to a 'Test Server' or sandbox where they can be tested for association, efficacy and consistency with existing or third party data sets, and to 'Other Members' of the system for analysis and expansion. The user interface 300 provides a powerful tool for analysing trait responses and aid understanding and compliance with wellness regimes.

From reading the present disclosure, other variations and modifications will be apparent to the skilled person. Such variations and modifications may involve equivalent and other features which are already known in the art of health regime development, and which may be used instead of, or in addition to, features already described herein.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality, a single processor or other unit may fulfil the functions of several means recited in the claims and reference signs in the claims shall not be construed as limiting the scope of the claims.

The following section comprises numbered clauses which are not claims but are additional statements of the invention.

1. A method of determining an optimum wellness regime that best meets one or more wellness goals of a user, said method comprising the steps of:

identifying one or more traits, said traits being one or more physiological, behavioural or biological states of the user that are potentially relevant to the wellness goals;
identifying one or more genetic factors of the user that are relevant to one or more of the traits;
identifying one or more environmental factors of the user that are relevant to said one or more of the traits;
assigning a weighting to each genetic and/or environmental factor for the traits, said weighting defining the likely effect that the genetic and environmental factors have on the traits;

calculating a trait score for each trait based on the weightings assigned to each trait, each trait score indicative of the likely biological, behavioural or physiological response of the user to the trait;

defining a trait profile for the user from the trait scores, said trait profile characterizing the user's likely biological, behavioural or physiological response to a wellness regime;

determining, based at least partly on the trait profile, a wellness regime that is optimised to the wellness goals of the user.

2. A method according to clause 1, wherein one or more of the traits comprise a plurality of trait bands, each trait band being indicative of particular trait outcome.

3. A method according to clause 2, wherein the step of calculating a trait profile further comprises the step of: assigning one or more of the traits to one of the trait bands depending on the relative strength and directionality of the trait score.

4. A method according to any preceding clause, wherein the step of determining a wellness regime further comprises the step of:
determining one or more recommendations based on the trait profile, said recommendations providing an actionable outcome for the user.

5. A method according to clause 4, wherein the recommendations are determined based on one or more of: trait scores; and/or trait profiles; and/or data elements, said data elements comprising one or more genetic factors, and/or one or more environmental factors and/or one or more wellness goals.

6. A method according to clause 4 or clause 5, wherein at least one of said recommendations comprises recommendation bands, said recommendation bands identifying a wellness action for the user, optimised towards meeting the wellness goals.

7. A method according to any preceding clause, wherein the traits include one or more of insulin sensitivity; obesity risk; gut microbiome profile; blood testosterone levels; dyslipidaemia, lactose intolerance, blood triglycerides level, blood glucose levels, oxidative muscle dominance, saturated fat level, satiety, folate metabolism, homocysteine levels, methionine levels, caffeine metabolism, hypertension levels, omega 6 intake or omega 3 to 6 ratio, circadian rhythm, sleep disturbance, trainable VO2 max, salt sensitivity, workout recovery between workout sessions, workout recovery during a workout session, lactate clearance levels, basal metabolism, lean body mass, endurance capability, power capability, conscious restraint, binge eating propensity, emotional eating propensity, eating behaviour and body fat.

8. A method according to any preceding clause, wherein the genetic factors are genetic variants.

9. A method according to clause 8, wherein the genetic variants include one or more: polymorphisms; and/or insertions; and/or deletions; and/or gene copy number variants.

10. A method according to any preceding clause, wherein the trait profile characterises biological systems of the user, said biological systems providing a representation of the user's physiological, behavioural and biological propensities and/or health status.

11. The method of clause 10, wherein the biological systems comprise a plurality of trait profiles, each trait profile defining one aspect of the user's biological system.

12. A method according to any preceding clause, wherein at least one genetic factor, and/or at least one environmental factor, are relevant to multiple traits.

13. A method according to any preceding clause, wherein the weighting assigned to the genetic factors, and/or the weighting assigned to the environmental factors, are different for different traits.

14. A method according to any preceding clause wherein the wellness regime is one or a combination of a nutrition plan, wellbeing plan, fat loss plan, muscle building plan, strength training plan, microbiotic supplementation plan, or a medical regime.

15. A method according to any preceding clause, wherein the wellness regime is selected from a database of known wellness regimes.

16. A method according to any preceding clause, wherein trait scores are calculated from the interaction between one or more genetic factors, between one or more environmental factors, and/or between a combination of genetic and environmental factors for each or a combination of traits.

17. A method according to any preceding clause, wherein separate trait scores are calculated for each separate trait or for combinations of traits.

18. A method according to clause 16 or clause 17, wherein the interaction is one or more of hierarchical, synergistic, additive, subtractive or antagonistic.

19. A method according to any preceding clause, wherein a trait score is modified by direct biophysical data.

20. A method according to clause 19, wherein the direct biophysical data is data defining the actual biological or physiological response of the user to one or more of said parameters.

21. A method according to clause 19 or clause 20, wherein the direct biophysical data is provided by a data interface, by a third party biophysical data system or by the user.

22. A method of any preceding clause wherein the step of determining a trait score comprises the step of: cross-referencing the one or more genetic factors and/or the one or more environmental factors relevant to said trait and calculating the trait score for each trait based on the cross-referencing.

23. A method of any preceding clause, wherein the step of calculating the trait profile comprises the steps of:

assigning each trait a default trait score indicating the average predicted response of an average user to said at least one variable; and
updating the default trait score with the trait score.

24. A method according to any preceding clause, wherein the trait profile is calculated from the hierarchical, synergistic, additive, subtractive or antagonistic interaction of the trait scores.

25. The method of any preceding clause, wherein the step of calculating a trait profile comprises the step of weighting the trait scores according to the wellness goals of the user.

26. The method of any preceding clause, wherein the weighting of a genetic or environmental factor for a trait is zero.

27. The method of any preceding clause, wherein the step of determining a wellness regime that is optimised for the user comprises the step of matching said trait profile to one or more reference trait profiles.

28. The method of clause 27, wherein the reference trait profiles have reference trait scores defining the reference response of a reference user to said traits.

29. The method of clause 27 or 28, wherein the reference trait profiles provide an overall reference response of a user having the genetic and environmental factors for the trait.

30. The method of any one of clauses 28 or clause 29, wherein the step of matching said trait profile includes the step of:
undertaking a statistical analysis of the overall predicted response of the user relative to the reference response.

31. The method of any preceding clause, wherein the trait profile suggests the user's propensities to phenotypic expressions.

32. The method of any preceding clause, wherein the genetic and environmental factors are scored according to ensemble research to determine a factor score indicative of the user's genetic and environmental factors compared to the ensemble research.

33. The method of clause 32, wherein the trait scores are modified using the factor score of each one or more genetic and environmental factor.

34. The method of any preceding clause, wherein the genetic and environmental factors are scored according to feedback from the and/or other users to determine a feedback score indicative of users feedback.

35. The method of any preceding clause, wherein the trait scores are modified using the feedback score of each genetic and environmental factor.

36. The method of any preceding clause, wherein the step of determining which wellness regime is optimised includes the steps of:

identifying a wellness goal of the user;
querying a database of traits relevant to wellness goals for the wellness goal; and
prioritising the traits identified by the database for the wellness goal.

37. The method of clause 36, wherein the prioritising comprises the steps of:

adjusting the trait scores of the user corresponding to the traits identified by the database according to weighting factors stored within the database; and
updating the trait profile based on the adjusted trait scores.

38. The method of any preceding clause, wherein the step of determining which wellness regime comprises the steps of:

cross-referencing the user's trait profile with trait profiles of other users of a databank of known wellness regimes; and
determining one or more optimum wellness regimes from the databank of wellness regimes, by selecting one or more known wellness regimes deemed most beneficial by users of similar trait profiles.

39. The method of clause 38, wherein the databank of wellness regimes are third party external wellness regimes.

40. The method of any preceding clause, wherein the user provides a feedback response regarding the wellness regime.

41. The method of any preceding clause, wherein the step of determining a wellness regime comprises the step of selecting a wellness regime based on previous user feedback responses.

42. The method of clause 41, wherein the user feedback is provided by an interfaced system, optionally a third party system.

43. The method of clause 40, 41 or clause 42, wherein the feedback response is collated within the or a databank of known health regimes.

44. The method of clause 40, 41 or clause 42, wherein the feedback response is used to update the weightings of one or more of the genetic or environmental factors.

45. The method of any one of clauses 40 to 44, wherein the trait score of the one or more traits is altered based on the feedback.

46. The method of clause 45, wherein the trait score is altered within a sandbox database until the overall predicted response of the user matches the feedback response.

47. The method of clause 46, wherein the trait values are supplied to the trait profile within a live database.

48. The method of any preceding clause, wherein the genetic factors, environmental factors and traits define a profile for the user.

49. The method of any preceding clause, wherein the genetic factors are identified from one or more of: a laboratory DNA test; a user DNA test; or medical laboratory data.

50. The method of any preceding clause, wherein the environmental factors are identified from one or more of: health and fitness applications; questionnaires; mobile applications; medical history; laboratory data; health and fitness monitors, user location, user psychological history, location conditions, or a computing device.

51. The method of any preceding clause, wherein the wellness regime is predicted using an artificial intelligence computing device.

52. The method of any preceding clause, further comprising the step of exporting information representative of said trait profile to a third-party server.

53. The method of clause 52, further comprising providing advertising to the user tailored to the information.

54. The method of any preceding clause, wherein the method further comprises the step of interfacing with external data sources on a third party server to supplement and/or incorporate user data within the trait profile.

55. The method of clause 54, wherein the interface is via a mobile application.

56. The method of any one of clauses 52 to 55, wherein the external data sources interface with a server implementing the method such that the server provides a guided manual for analysis of external data.

57. A system for carrying out the method of any preceding clause.

58. A computer readable medium containing instructions that, when read by a processor, cause that processor to implement a method according to any preceding method clause.

## Claims

1. A method of determining an optimum wellness regime that best meets one or more wellness goals of a user, said method comprising the steps of:

    identifying one or more traits, said traits being one or more physiological, behavioural or biological states of the user that are potentially relevant to the wellness goals;
    identifying one or more genetic factors of the user that are relevant to one or more of the traits;
    identifying one or more environmental factors of the user that are relevant to said one or more of the traits;
    assigning a weighting to each genetic and/or environmental factor for the traits, said weighting defining the likely effect that the genetic and environmental factors have on the traits;
    calculating a trait score for each trait based on the weightings assigned to each trait, each trait score indicative of the likely biological, behavioural or physiological response of the user to the trait;
    defining a trait profile for the user from the trait scores, said trait profile characterizing the user's likely biological, behavioural or physiological response to a wellness regime;
    determining, based at least partly on the trait profile, a wellness regime that is optimised to the wellness goals of the user.

2. A method according to claim 1, wherein:

    a) one or more of the traits comprise a plurality of trait bands, each trait band being indicative of particular trait outcome optionally wherein the step of calculating a trait profile further comprises the step of:
    assigning one or more of the traits to one of the trait bands depending on the relative strength and directionality of the trait score; and/or
    b) the step of determining a wellness regime further comprises the step of:
    determining one or more recommendations based on the trait profile, said recommendations providing an actionable outcome for the user, optionally, wherein the recommendations are determined based on one or more of: trait scores; and/or trait profiles; and/or data elements, said data elements comprising one or more genetic factors, and/or one or more environmental factors and/or one or more wellness goals, and/or

c) at least one of said recommendations comprises recommendation bands, said recommendation bands identifying a wellness action for the user, optimised towards meeting the wellness goals.

3. A method according to any preceding claim, wherein: a) the traits include one or more of insulin sensitivity; obesity risk; gut microbiome profile; blood testosterone levels; dyslipidaemia, lactose intolerance, blood triglycerides level, blood glucose levels, oxidative muscle dominance, saturated fat level, satiety, folate metabolism, homocysteine levels, methionine levels, caffeine metabolism, hypertension levels, omega 6 intake or omega 3 to 6 ratio, circadian rhythm, sleep disturbance, trainable VO2 max, salt sensitivity, workout recovery between workout sessions, workout recovery during a workout session, lactate clearance levels, basal metabolism, lean body mass, endurance capability, power capability, conscious restraint, binge eating propensity, emotional eating propensity, eating behaviour and body fat; and/or

b) the genetic factors are genetic variants,
optionally wherein the genetic variants include one or more: polymorphisms; and/or insertions; and/or deletions; and/or gene copy number variants; and/or
c) the trait profile characterises biological systems of the user, said biological systems providing a representation of the user's physiological, behavioural and biological propensities and/or health status optionally wherein the biological systems comprise a plurality of trait profiles, each trait profile defining one aspect of the user's biological system; and/or
d) at least one genetic factor, and/or at least one environmental factor, are relevant to multiple traits; and/or
e) the weighting assigned to the genetic factors, and/or the weighting assigned to the environmental factors, are different for different traits; and/or
f) the wellness regime is one or a combination of a nutrition plan, wellbeing plan, fat loss plan, muscle building plan, strength training plan, microbiotic supplementation plan, or a medical regime; and/or
g) the wellness regime is selected from a database of known wellness regimes; and/or
h) trait scores are calculated from the interaction between one or more genetic factors, between one or more environmental factors, and/or between a combination of genetic and environmental factors for each or a combination of traits; and/or
i) separate trait scores are calculated for each separate trait or for combinations of traits.

4. A method according to claim 3h) or claim 3i), wherein the interaction is one or more of hierarchical, synergistic, additive, subtractive or antagonistic.

5. A method according to any preceding claim, wherein a trait score is modified by direct biophysical data,

optionally wherein the direct biophysical data is data defining the actual biological or physiological response of the user to one or more of said parameters; and/or optionally
wherein the direct biophysical data is provided by a data interface, by a third party biophysical data system or by the user.

6. A method of any preceding claim wherein:

a) the step of determining a trait score comprises the step of: cross-referencing the one or more genetic factors and/or the one or more environmental factors relevant to said trait and calculating the trait score for each trait based on the cross-referencing; and/or
b) the step of calculating the trait profile comprises the steps of:

assigning each trait a default trait score indicating the average predicted response of an average user to said at least one variable; and
updating the default trait score with the trait score; and/or

c) the trait profile is calculated from the hierarchical, synergistic, additive, subtractive or antagonistic interaction of the trait scores; and/or
d) the step of calculating a trait profile comprises the step of weighting the trait scores according to the wellness goals of the user; and/or
e) the weighting of a genetic or environmental factor for a trait is zero.

7. The method of any preceding claim, wherein the step of determining a wellness regime that is optimised for the user comprises the step of matching said trait profile to one or more reference trait profiles, optionally wherein: a) the reference trait profiles have reference trait scores defining the reference response of a reference user to said traits; and/or

b) the reference trait profiles provide an overall reference response of a user having the genetic and environmental factors for the trait.

8. The method of claim 7a) or 7b), wherein the step of matching said trait profile includes the step of: undertaking a statistical analysis of the overall predicted response of the user relative to the reference response.

9. The method of any preceding claim, wherein:

a) the trait profile suggests the user's propensities to phenotypic expressions; and/or
b) the genetic and environmental factors are scored according to ensemble research to determine a factor score indicative of the user's genetic and environmental factors compared to the ensemble research optionally wherein the trait scores are modified using the factor score of each one or more genetic and environmental factor; and/or
c) the genetic and environmental factors are scored according to feedback from the and/or other users to determine a feedback score indicative of users feedback; and/or
d) the trait scores are modified using the feedback score of each genetic and environmental factor; and/or
e) the step of determining which wellness regime is optimised includes the steps of:

identifying a wellness goal of the user;
querying a database of traits relevant to wellness goals for the wellness goal; and
prioritising the traits identified by the database for the wellness goal, optionally, wherein the prioritising comprises the steps of:

adjusting the trait scores of the user corresponding to the traits identified by the database according to weighting factors stored within the database; and
updating the trait profile based on the adjusted trait scores; and/or

f) the step of determining which wellness regime comprises the steps of:

cross-referencing the user's trait profile with trait profiles of other users of a databank of known wellness regimes; and
determining one or more optimum wellness regimes from the databank of wellness regimes, by selecting one or more known wellness regimes deemed most beneficial by users of similar trait profiles, optionally wherein the databank of wellness regimes are third party external wellness regimes.

10. The method of any preceding claim, wherein:

a) the user provides a feedback response regarding the wellness regime; or
b) the step of determining a wellness regime comprises the step of selecting a wellness regime based on previous user feedback responses, optionally

wherein the user feedback is provided by an interfaced system, optionally a third party system.

11. The method of claim 10, wherein:

a) the feedback response is collated within the or a databank of known health regimes; or
b) the feedback response is used to update the weightings of one or more of the genetic or environmental factors; and/or
c) the trait score of the one or more traits is altered based on the feedback, optionally

wherein the trait score is altered within a sandbox database until the overall predicted response of the user matches the feedback response, further optionally, wherein the trait values are supplied to the trait profile within a live database.

12. The method of any preceding claim, wherein:

a) the genetic factors, environmental factors and traits define a profile for the user; and/or

b) the genetic factors are identified from one or more of: a laboratory DNA test; a user DNA test; or medical laboratory data; and/or

c) the environmental factors are identified from one or more of: health and fitness applications; questionnaires; mobile applications; medical history; laboratory data; health and fitness monitors, user location, user psychological history, location conditions, or a computing device; and/or

d) the wellness regime is predicted using an artificial intelligence computing device; and/or

e) the method further comprises the step of exporting information representative of said trait profile to a third-party server, optionally, further comprising providing advertising to the user tailored to the information; and/or

f) the method further comprises the step of interfacing with external data sources on a third party server to supplement and/or incorporate user data within the trait profile optionally wherein the interface is via a mobile application.

13. The method of any one of claims 12e) or f), wherein the external data sources interface with a server implementing the method such that the server provides a guided manual for analysis of external data.

14. A system for carrying out the method of any preceding claim.

15. A computer readable medium containing instructions that, when read by a processor, cause that processor to implement a method according to any preceding method claim.

Figure 1

Figure 2

Figure 3

EP 4 280 225 A2

| Stage 1 | How do I lose fat? |
|---------|--------------------|

| Stage 2 | Collect Search Methods |
|---------|------------------------|

| Stage 3 | Interrogate question database to determine relevant traits and systems to assess |
|---------|--------------------|

Stage 3A

| 1 | How do i lose fat? | |
|---|--------------------|---|
| 2 | 2 | |
| 3 | Relevant Categories | Relevant Traits |
| 4 | Daily caloric intake | A,B |
| 5 | Amount/Carbohydrate | A,C,D |
| 6 | Type/Carbohydrate | |

| Stage 4 | Interrogate trait location database to determine where relevant traits and their scores are stored |
|---------|--------------------|

Stage 4A

| | A | B | |
|---|-----|-----------|---|
| 1 | How do I lose fat? | | |
| 2 | | | |
| 3 | Trait | Location | |
| 4 | A | Cell B2 | |
| 5 | B | Cell B3 | |
| 6 | C | Cell B4 | |
| 7 | D | Cell B5 | |

## Figure 4a

EP 4 280 225 A2

## Stage 5

Interrogate rules database for weightings, hierarchies and interactions

Weightings | Interactions

Stage 5A — Trait C is 10x more important in the scenario | Trait A is irrelevant if trait D is greater than 10 — Stage 5B

Stage 5A1 — B4 X 10 | if B5 > 10, do not use B2 — Stage 5B1

Stage 6 — Compile data collected from search methods

Stage 7 — Collect relevant trait scores

Matched by Algorithm

Stage 8 — Apply goal specific weightings and interactions

### User Profile

|  | A | B |
|---|---|---|
| 1 | Trait | User Trait Score |
| 2 | Trait A | 3 |
| 3 | Trait B | 4 |
| 4 | Trait C | 7 |
| 5 | Trait D | 11 |
| 6 | Trait E | 5 |
| 7 | Trait F | 15 |

Interpreted//Modified trait scores

|  | A | B |
|---|---|---|
| 1 | Trait | Weighted Trait Score |
| 2 | A | 0 |
| 3 | B | 3 |
| 4 | C | 70 |
| 5 | D | 11 |

Stage 9

## Figure 4b

35

| | Stage 10 | Interrogate database of predefined fitness and diet plans using interpreted/modified trait scores |
|---|---|---|

**Stage 10** — Interrogate database of predefined fitness and diet plans using interpreted/modified trait scores

**Stage 11** — Fetch user plans from database of predfined fitness and diet plans

**Stage 12** — Output Diet and Fitness plan to user

| | A | B | C |
|---|---|---|---|
| 1 | How do I lose fat? | | |
| 2 | | | |
| 3 | Result Set | Trait Score Boundaries | Recommended Diet and Fitness plans |
| 4 | 1 | if A<5, B<10, C<100, D>5 | Diet 1 and fitness plan 8 |
| 5 | 2 | if A<15, B<12, C<40, D>3 | Diet 7 and fitness plan 4 |
| 6 | 3 | if A>10, B>0, C<5, D=0 | Diet 2 and fitness plan 2 |
| 7 | 4 | if A>100, B>100, C>50, D>10 | Do not progress |

# Figure 4c

Figure 5

EP 4 280 225 A2

| BLOOD_FAT | BLOOD_GLUC |
|---|---|

312 310 300

Rules ～320 [Add]

| Name | Gender | Next | |
|---|---|---|---|
| Blood Glucose Measurement: | Both ▽ | Exit ▽ | × |

322 324 326

Condition Sets ～330 [Add]

| Name | Operator | Rule Value | |
|---|---|---|---|
| Blood Glucose Measurement: | Subtract ▽ | 7.5 ▽ | × |

332 334 336

Conditions ～340 [Add]

| Clause | Data Element | Check For | Value 1 | Value 2 | Value | |
|---|---|---|---|---|---|---|
| Where ▽ | TEST_BLOOD_GLUCOSE ▽ | Between ▽ | 125 ▽ | 1000 | | × |

342 344 346 347 348

Figure 6